# EUROPÄISCHE PATENTSCHRIFT

(11) **EP 1 339 789 B1**
(45) Veröffentlichungstag und Bekanntmachung des Hinweises auf die Patenterteilung: **16.02.2005**
(21) Anmeldenummer: 01985829.9
(22) Anmeldetag: 28.11.2001
(51) Int. Cl.: C08L 33/00, A61K 7/48, A61K 7/06

(54) **ELEKTROLYTHALTIGE KOSMETISCHE, PHARMAZEUTISCHE UND DERMATOLOGISCHE MITTEL**
COSMETIC, PHARMACEUTICAL AND DERMATOLOGICAL PRODUCTS CONTAINING AN ELECTROLYTE
AGENTS COSMETIQUES, PHARMACEUTIQUES ET DERMATOLOGIQUES ELECTROLYTIQUES

(30) Priorität: 01.12.2000 DE 10059824
(43) Veröffentlichungstag der Anmeldung: 03.09.2003
(73) Patentinhaber: Clariant GmbH, 65929 Frankfurt am Main (DE)
(72) Erfinder: LÖFFLER, Matthias, 65527 Niedernhausen (DE); MORSCHHÄUSER, Roman, 55122 Mainz (DE)
(74) Vertreter: Paczkowski, Marcus, Dr.
(86) Internationale Anmeldenummer: PCT/EP2001/013868
(87) Internationale Veröffentlichungsnummer: WO 2002/044267

(56) Entgegenhaltungen:
- EP-A- 1 026 219
- WO-A-01/96422
- WO-A1-00/07638
- US-A- 6 123 960

## Beschreibung

Die vorliegende Erfindung betrifft Elektrolythaltige kosmetische, pharmazeutische und dermatologische Mittel, enthaltend kammförmige Copolymere auf Basis von Acryloyldimethyltaurinsäure.

Polymere auf Basis von Acryloyldimethyltaurinsäure sind bereits bekannt. Beispielsweise werden in der WO 00/07638 Polymere beschrieben, die unter Verwendung von Acryloyldimethyltaurinsäure oder ihrer Salze hergestellt werden können. US 6,123,960 offenbart vernetzte Poly(2-acrylamido-2-methylpropansulfonsäuren), die zu mindestens 90 % neutralisiert sind.

Verbraucherwünsche und Rheologie kosmetischer Produkte sind eng miteinander verknüpft. So wird z.B. das visuelle Erscheinungsbild einer Creme oder Lotion durch die Viskosität beeinflusst. Die sensorischen Eigenschaften, wie Konsistenz oder Verteilbarkeit bestimmen das individuelle Profil eines Kosmetikproduktes. Auch die Lagerstabilität der Formulierung, z.B von perlglänzenden Shampoos oder ölhaltigen Duschbädern steht in enger Abhängigkeit zu den rheologischen Eigenschaften des Produktes.

Ein flüssiges Tensidsystem muss zudem auch eine Viskosität haben, die dem jeweiligen Verwendungszweck angepasst ist und sich möglichst variabel einstellen lässt. So ist die Viskosität ein entscheidendes Kriterium für die Qualität eines flüssigen Tensidpräparates. Es werden z.B. von einem Duschgel relativ hohe Viskositäten gefordert, während ein Haarshampoo gewöhnlich eine fließfähige Flüssigkeit mit einer relativ niedrigen Viskosität darstellt.

Im kosmetischen Bereich kommt daher Polyelektrolyten als Verdicker und Gelbildner eine tragende Rolle zu. Stand der Technik sind insbesondere die auf Basis der Poly(meth)acrylsäure hergestellten Polyacrylsäuren und deren wasserlösliche Copolymere. Die Vielfalt der möglichen Strukturen und die damit verbundenen vielfältigen Anwendungsmöglichkeiten drücken sich nicht zuletzt in einer Vielzahl von neuen Patenten aus, die seit Mitte der 70iger Jahre weltweit angemeldet wurden. Ein wesentlicher Nachteil dieser Verdicker auf Basis Poly(meth)acrylsäure ist die starke pH-Abhängigkeit der Verdickungsleistung. So wird im allgemeinen Viskosität nur dann aufgebaut, wenn der pH Wert der Formulierung oberhalb von pH 6 eingestellt ist und somit die Poly(meth)acrylsäure in neutralisierter Form vorliegt. Ferner sind die entsprechenden Gele/Formulierung empfindlich gegenüber UV-Strahlung als auch Scherung und vermitteln auf der Haut zudem ein klebriges Gefühl. Auch die Handhabung dieser Verdickerpolymere ist verbesserungswürdig: Da die Verdicker auf Basis von Poly(meth)acrylsäure i.a. in saurer Form vorliegen, bedarf es bei der Formulierung eines zusätzlichen Neutralisationsschrittes. Ein weiterer gravierender Nachteil der Verdicker auf Basis von Poly(meth)acrylsäure ist eine die starke Empfindlichkeit gegenüber Elektrolyten. Sobald wässrige Systeme (Gele, Emulsionen etc), enthaltend Polyelektrolyte, in Kontakt mit Salz kommen, fällt die Viskosität dramatisch ab. Dieser Viskositätsabbau wird durch eine Abschirmung der ionischen Zentren im Polymer durch den Elektrolyt erklärt, was zu einem Kollabieren der Polymer-Netzwerkstruktur führt. Zudem werden die wässrige Systeme, enthaltend Polyelektrolyte, bei Kontakt mit Salz trüb.

Verdickung von elektrolythaltigen wässrigen Tensidsystemen erfolgt daher im allgemeinen durch Zugabe von Cellulose Derivate, natürlichen Polymeren (z.B. Xanthan Gum, Guar), nichtionischen Tensiden oder Polyethylenglykol-Derivaten.

Alle diese Verdickersysteme führen zu Tensidsystemen, die strukturviskos und/oder thixotrop sind. Je nach Verdicker sind allerdings auch diverse Nachteile in Kauf zu nehmen:
Die Verdickungsleistung von vielen Cellulose Derivaten wird durch Salz stark herabgesetzt.
Natürliche Polymere sind sehr schwierig zu verarbeiten, zudem sind klare Formulierungen häufig nicht realisierbar.
Bekannte nichtionische Verdickungsmittel für flüssige Tensidformulierungen sind unter anderem Fettsäurealkanolamide. Als Fettsäurealkanolamid wird in der Praxis bevorzugt das Kokosfettsäurediethanolamid eingesetzt. Es zeigt gegenüber anderen Fettsäurediethanolamiden die besten Verdickungseigenschaften. Nachteilig ist jedoch das Vorhandensein von Aminischen NebenverbindungenNerunreinigungen.
Polyethylenglykol-Derivate wie PEG 6000 Distearate, PEG-120 Methyl Glucose Dioleate, PEG-150 Pentaerythrityl Tetrastearate, PEG-20 Methyl Glucose Sesquistearate etc, sind sowohl aufwendig in der Herstellung (mangelnde Konformität von batch zu batch), als auch teilweise kompliziert zu verarbeiten (Aufschmelzen bzw. Lösen bei hoher Temperatur, hohe Einsatzmengen).

Somit besteht ein Bedarf für Polymere, die hervorragende Verdickerleistung auch in Gegenwart von Elektrolyten gewährleisten, einfach zu verarbeiten sind, über einen breiten pH Bereich einsetzbar sind und hervorragende rheologische und sensorische Eigenschaften bei gleichzeitig guter Stabilität zeigen. Es stellte sich zudem die Aufgabe, Zusätze zu finden, die sich zur Viskositätseinstellung in Tensidformulierungen eignen, vorzugsweise klare Lösungen geben, universal und bei geringer Einsatzkonzentration verwendbar sind, und zudem toxikologisch unbedenklich sind.

Überraschend wurde nun gefunden, dass eine neue Klasse von kammförmigen Copolymeren auf Basis von Acryloyldimethyltaurinsäure- die sowohl als Verdicker, Konsistenzgeber, Emulgator, Dispergator, Gleitmittel, Conditioner und/oder Stabilisator geeignet sind - hervorragend zur Verwendung in elektrolythaltigen kosmetischen, pharmazeutischen und dermatologischen Mitteln geeignet sind.

Gegenstand der Erfindung sind daher elektrolythaltige kosmetische, dermatologische und pharmazeutische Mittel, enthaltend mindestens ein Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt, und wobei mindestens ein Makromonomer ein Makromonomer gemäß Formel (IV) 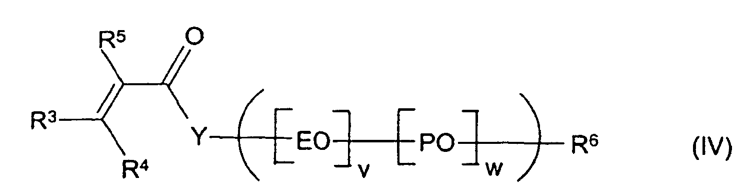 ist, worin
   R₃, R₄, R₅ und R₆ unabhängig voneinander Wasserstoff oder n-aliphatische, iso-aliphatische, olefinische, cycloaliphatische, arylaliphatische oder aromatische (C₁-C₃₀)-Kohlenwasserstoffreste bedeuten,
   v und w unabhängig voneinander 0 bis 500 sind,
   die Summe aus v und w im Mittel ≥1 ist, und
   Y eine Brücke ausgewählt aus -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)- bedeutet,
G) wobei die Copolymerisation gegebenenfalls in Gegenwart mindestens eines polymeren Additivs mit zahlenmittleren Molekulargewichten von 200 g/mol bis 10⁹ g/mol erfolgt,
H) mit der Maßgabe, dass die Komponente A) mit mindestens einer Komponente ausgewählt aus einer der Gruppen D) bis G) copolymerisiert wird,
und wobei der Gehalt an Acryloyldimethyltaurinsäure bzw. Acryloyldimethyltauraten bezogen auf die Gesamtmasse der Copolymere mindestens 0,1 Gew.-% beträgt.

Die erfindungsgemäßen Copolymere besitzen bevorzugt ein Molekulargewicht von 10³ g/mol bis 10⁹ g/mol, besonders bevorzugt von 10⁴ bis 10⁷ g/mol, insbesondere bevorzugt 5*10⁴ bis 5*10⁶ g/mol.

Bei den Acryloyldimethyltauraten kann es sich um die anorganischen oder organischen Salze der Acryloyldimethyltaurinsäure handeln. Bevorzugt werden die Li⁺-, Na⁺-, K⁺-, Mg⁺⁺-, Ca⁺⁺-, Al⁺⁺⁺- und/oder NH₄⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um (C₁-C₂₂)-Alkylreste oder (C₂-C₁₀)-Hydroxyalkylreste handeln kann. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreter im Sinne der Erfindung sind.

Der Neutralisationsgrad der Acryloyldimethyltaurinsäure kann zwischen 0 und 100 % betragen, besonders bevorzugt ist ein Neutralisationsgrad von oberhalb 80%.

Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-%.

Als Comonomere B) können alle olefinisch ungesättigten, nicht kationischen Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.
Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 30.
Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.
Als Gegenionen bevorzugt sind Li⁺, Na⁺, K⁺, Mg⁺⁺, Ca⁺⁺, Al⁺⁺⁺, NH₄⁺, Monoalkylammonium-, Dialkylammonium-, Trialkylammonium- und/oder Tetraalkylammoniumreste, wobei es sich bei den Alkylsubstituenten der Amine unabhängig voneinander um (C₁-C₂₂)-Alkylreste oder (C₂-C₁₀)-Hydroxyalkylreste handeln kann. Zusätzlich können auch ein bis dreifach ethoxylierte Ammoniumverbindungen mit unterschiedlichem Ethoxylierungsgrad Anwendung finden. Der Neutralisationsgrad der Carbonsäuren kann zwischen 0 und 100 % betragen.
Als Comonomere B) weiterhin bevorzugt sind offenkettige N-Vinylamide, bevorzugt N-Vinylformamid (VIFA), N-Vinylmethylformamid, N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethylacrylamid, N,N-Diethylacrylamid und N,N-Diisopropylacrylamid; alkoxylierte Acryl- und Methacrylamide, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester]; N,N-Dimethylaminomethacrylat; Diethylamino-methylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen.
Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.
Der Gewichtsanteil der Comonomere B), bezogen auf die Gesamtmasse der Copolymere, kann 0 bis 99,8 Gew.-% betragen und beträgt bevorzugt 0,5 bis 80 Gew.-%, besonders bevorzugt 2 bis 50 Gew.-%.

Als Comonomere C) kommen alle olefinisch ungesättigten Monomere mit kationischer Ladung in Frage, die in der Lage sind, in den gewählten Reaktionsmedien mit Acryloyldimethyltaurinsäure oder deren Salze Copolymere zu bilden. Die dabei resultierende Verteilung der kationischen Ladungen über die Ketten hinweg kann statistisch, alternierend, block- oder gradientenartig sein. Es sei darauf hingewiesen werden, dass unter den kationischen Comonomeren C) auch solche zu verstehen sind, die die kationische Ladung in Form einer betainischen, zwitterionischen, oder amphoteren Struktur tragen.
Comonomere C) im Sinne der Erfindung sind auch aminofunktionalisierte Precursor, die durch polymeranaloge Reaktionen in Ihre entsprechenden quaternären (z.B. Reaktion mit Dimethylsulfat, Methylchlorid), zwitterionischen (z.B. Reaktion mit Wasserstoffperoxid), betainischen (z.B. Reaktion mit Chloressigsäure), oder amphotere Derivate überführt werden können.

Besonders bevorzugt als Comonomere C) sind
Diallyldimethylammoniumchlorid (DADMAC),
[2-(Methacryloyloxy)ethyl]trimethylammoniumchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain.

Der Gewichtsanteil der Comonomeren C) kann, bezogen auf die Gesamtmasse der Copolymere, 0,1 bis 99,8 Gew.-%, bevorzugt 0,5 bis 30 Gew.-% und insbesondere bevorzugt 1 bis 20 Gew.-% betragen.

Als polymerisationsfähige, siliziumhaltige Komponenten D) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen silikonhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Komponenten mit zwei oder mehr polymerisationsaktiven Gruppen führt zum Aufbau verzweigter oder vernetzter Strukturen.

Bevorzugte silikonhaltige Komponenten sind solche gemäß Formel (I)

R¹-Z-[(Si(R³R⁴)-O-)_{w}-(Si(R⁵R⁶)-O)ₓ-]-R² (I).

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ einen Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest dar.
Zur Anbindung der silikonhaltigen Polymerkette an die reaktive Endgruppe R¹ ist eine geeignete chemische Brücke Z erforderlich. Bevorzugte Brücken Z sind -O-, -((C₁ - C₅₀)Alkylen)-, -((C₆ - C₃₀) Arylen)-, -((C₅ - C₈) Cycloalkylen)-, -((C₁-C₅₀)Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann. Weiterhin geeignet als Brückegruppierungen Z sind -((C₁ - C₁₀)Alkyl)-(Si(OCH₃)₂)- und -(Si(OCH₃)₂)-.
Der polymere Mittelteil wird durch silikonhaltige Wiederholungseinheiten repräsentiert.
Die Reste R³, R⁴, R⁵ und R⁶ bedeuten unabhängig voneinander -CH₃, -O-CH₃, -C₆H₅ oder -O-C₆H₅.
Die Indizes w und x repräsentieren stöchiometrische Koeffizienten, die unabhängig voneinander 0 bis 500, bevorzugt 10 bis 250, betragen.
Die Verteilung der Wiederholungseinheiten über die Kette hinweg kann nicht nur rein statistisch, sondern auch blockartig, alternierend oder gradientenartig sein kann.
R² kann einerseits einen aliphatischen, olefinischen, cycloaliphatischen, arylaliphatischen oder aromatischen (C₁ - C₅₀)-Kohlenwasserstoffrest symbolisieren (linear oder verzweigt) oder -OH, -NH₂, -N(CH₃)₂, -R⁷ oder für die Struktureinheit [-Z-R¹] stehen. Die Bedeutung der beiden Variablen Z und R¹ wurde bereits erklärt. R⁷ steht für weitere Si-haltige Gruppierungen. Bevorzugte R⁷-Reste sind -O-Si(CH₃)₃, -O-Si(Ph)₃, -O-Si(O-Si(CH₃)₃)₂CH₃) und -O-Si(O-Si(Ph)₃)₂Ph).
Wenn R² ein Element der Gruppe [-Z-R¹] darstellt, handelt es sich um difunktionelle, Monomere, die zur Vernetzung der entstehenden Polymerstrukturen herangezogen werden können.
Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Besonders bevorzugte silikonhaltige Komponenten sind die folgenden acrylischoder methacrylisch modifizierten silikonhaltigen Komponenten:

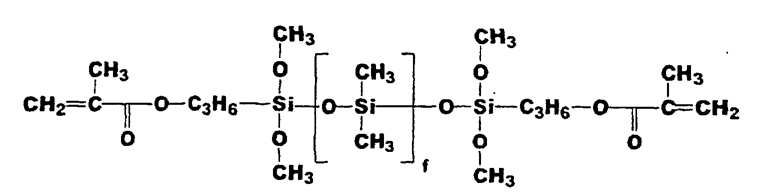

Methacryloxypropyldimethylsilyl endgeblockte Polydimethylsiloxane (f=2 bis 500)

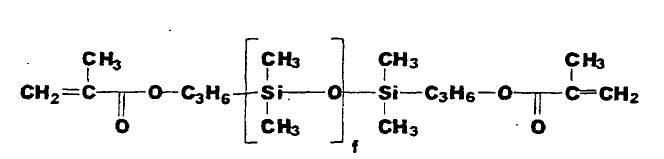

Methacryloxypropyl endgeblockte Polydimethylsiloxane (f= 2 bis 500 bis)

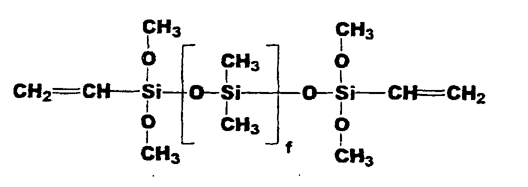

Vinyldimethoxysilyl endgeblockte Polydimethylsiloxane (f=2-500).

Bezogen auf die Gesamtmasse der Copolymere kann der Gehalt an siliziumhaltigen Komponenten bis 99,8 Gew.-%, bevorzugt 0,5 bis 30 Gew.-%, besonders bevorzugt 1 bis 20 Gew.-% betragen.

Als polymerisationsfähige, fluorhaltige Komponenten E) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen fluorhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen, fluorhaltigen Komponenten E) ist auch möglich, wobei dem Experten klar ist, dass monofunktionelle Vertreter zur Bildung kammförmiger Strukturen führen, wohingegen di-, tri-, oder polyfunktionelle Komponenten E) zu zumindest teilvernetzten Strukturen führen.

Bevorzugte fluorhaltige Komponenten E) sind solche gemäß Formel (II)

R¹-Y-CᵣH₂ᵣCₛF₂ₛCF₃ (II)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ ein Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest, besonders bevorzugt einen Acryl- und Methacrylrest, dar.
Zur Anbindung der fluorhaltigen Gruppierung an die reaktive Endgruppe R¹ ist eine geeignete chemische Brücke Y erforderlich. Bevorzugte Brücken Y sind -O-, -C(O)-, -C(O)-O-, -S-, -O-CH₂-CH(O-)-CH₂OH, -O-CH₂-CH(OH)-CH₂-O-, -O-SO₂-O- , -O-S(O)-O-, -PH-, -P(CH₃)-, -PO₃₋, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten und die Verteilung der EO- und PO-Einheiten statistisch oder blockförmig sein kann.
Bei r und s handelt es sich um stöchiometrische Koeffizienten, die unabhängig voneinander Zahlen von 0 bis 200 bedeuten.

Bevorzugte fluorhaltige Komponenten E) gemäß Formel (II) sind
Perfluorhexylethanol-methacrylat,
Perfluorhexoylpropanol-methacrylat,
Perfluoroctyethanol-methacrylat,
Perfluoroctylpropanol-methacrylat,
Perfluorhexylethanolylpolygycolether-methacrylat,
Perfluorhexoyl-propanolyl-poly-[ethylglykol-co-propylenglycolether]-acrylat,
Perfluoroctyethanolyl-poly-[ethylglykol-blockco-propylenglycolether]-methacrylat,
Perfluoroctylpropanolyl-polypropylen-glycolether-methacrylat.

Bezogen auf die Gesamtmasse des Copolymeren kann der Gehalt an fluorhaltigen Komponenten bis 99,9 Gew.-%, bevorzugt 0,5 bis 30 Gew.-%, besonders bevorzugt 1 bis 20 Gew.-% betragen.

Bei den Makromonomeren F) handelt sich um mindestens einfach olefinisch funktionalisierte Polymere mit einer oder mehreren diskreten Wiederholungseinheiten und einem zahlenmittleren Molekulargewicht größer oder gleich 200 g/mol. Bei der Copolymerisation können auch Mischungen chemisch unterschiedlicher Makromonomere F) eingesetzt werden. Bei den Makromonomeren handelt es sich um polymere Strukturen; die aus einer oder mehreren Wiederholungseinheit(en) aufgebaut sind und eine für Polymere charakteristische Molekulargewichtsverteilung aufweisen.

Bevorzugt als Makromonomere F) sind Verbindungen gemäß Formel (III)

R¹ - Y - [(A)ᵥ - (B)_{w} - (C)ₓ - (D)_{z}] - R² (III)

R¹ stellt eine polymerisationsfähige Funktion aus der Gruppe der vinylisch ungesättigten Verbindungen dar, die zum Aufbau polymerer Strukturen auf radikalischem Wege geeignet sind. Bevorzugt stellt R¹ einen Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest dar.
Zur Anbindung der Polymerkette an die reaktive Endgruppe ist eine geeignete yerbrückende Gruppe Y erforderlich. Bevorzugte Brücken Y sind -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)-, besonders bevorzugt -O-.
Der polymere Mittelteil des Makromonomeren wird durch die diskreten Wiederholungseinheiten A, B, C und D repräsentiert. Bevorzugte Wiederholungseinheiten A,B,C und D leiten sich ab von Acrylamid, Methacrylamid, Ethylenoxid, Propylenoxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid.
Die Indizes v, w, x und z in Formel (III) repräsentieren die stöchiometrische Koeffizienten betreffend die Wiederholungseinheiten A, B, C und D. v, w, x und z betragen unabhängig voneinander 0 bis 500, bevorzugt 1 bis 30, wobei die Summe der vier Koeffizienten im Mittel ≥ 1 sein muss.
Die Verteilung der Wiederholungseinheiten über die Makromonomerkette kann statistisch, blockartig, alternierend oder gradientenartig sein.
R² bedeutet einen linearen oder verzweigten aliphatischen, olefinischen, cycloaliphatischen, arylaliphatischen oder aromatischen (C₁-C₅₀)-Kohlenwasserstoffrest, OH, -NH₂, -N(CH₃)₂ oder ist gleich der Struktureinheit [-Y-R¹].
Im Falle von R² gleich [-Y-R¹] handelt es sich um difunktionelle Makromonomere, die zur Vernetzung der Copolymere geeignet sind.
Besonders bevorzugt als Makromonomere F) sind acrylisch- oder methacrylisch monofunktionalisierte Alkylethoxylate gemäß Formel (IV). 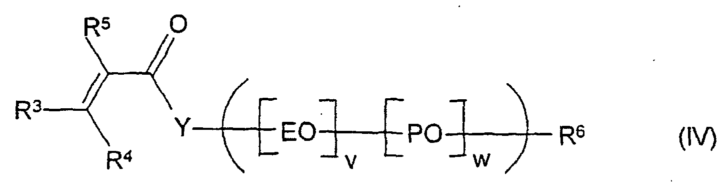 R₃, R₄, R₅ und R₆ bedeuten unabhängig voneinander Wasserstoff oder n-aliphatische, iso-aliphatische, olefinische, cycloaliphatische, arylaliphatische oder aromatische (C₁-C₃₀)-Kohlenwasserstoffreste.
Bevorzugt sind R₃ und R₄ gleich H oder -CH₃, besonders bevorzugt H; R₅ ist gleich H oder -CH₃; und R₆ ist gleich einem n-aliphatischen, iso-aliphatischen, olefinischen, cycloaliphatischen, arylaliphatischen oder aromatischen (C₁-C₃₀)-Kohlenwasserstoffrest.
v und w sind wiederum die stöchiometrischen Koeffizienten betreffend die Ethylenoxideinheiten (EO) und Propylenoxideinheiten (PO). v und w betragen unabhängig voneinander 0 bis 500, bevorzugt 1 bis 30, wobei die Summe aus v und w im Mittel ≥ 1 sein muss. Die Verteilung der EO- und PO-Einheiten über die Makromonomerkette kann statistisch, blockartig, alternierend oder gradientenartig sein. Y steht für die obengenannten Brücken.

Weiterhin insbesondere bevorzugte Makromonomeren F) haben die folgende Struktur gemäß Formel (IV):

| Bezeichnung | R³ | R⁴ | R⁵ | R⁶ | v | w |
|---|---|---|---|---|---|---|
| ®LA-030-methyacrylat | H | H | -CH₃ | -Lauryl | 3 | 0 |
| ®LA-070-methacrylat | H | H | -CH₃ | -Lauryl | 7 | 0 |
| ®LA-200-methacrylat | H | H | -CH₃ | -Lauryl | 20 | 0 |
| ®LA-250-methacrylat | H | H | -CH₃ | -Lauryl | 25 | 0 |
| ®T-080-methyacrylat | H | H | -CH₃ | -Talk | 8 | 0 |
| ®T-080-acrylat | H | H | H | -Talk | 8 | 0 |
| ®T-250-methyacrylat | H | H | -CH₃ | -Talk | 25 | 0 |
| ®T-250-crotonat | -CH₃ | H | -CH₃ | -Talk | 25 | 0 |
| ®OC-030-methacrylat | H | H | -CH₃ | -Octyl | 3 | 0 |
| ®OC-105-methacrylat | H | H | -CH₃ | -Octyl | 10 | 5 |
| ®Behenyl-010-methyaryl | H | H | H | -Behenyl | 10 | 0 |
| ®Behenyl-020-methyaryl | H | H | H | -Behenyl | 20 | 0 |
| ®Behenyl-010-senecionyl | -CH₃ | -CH₃ | H | -Behenyl | 10 | 0 |
| ®PEG-440-diacrylat | H | H | H | -Acryl | 10 | 0 |
| ®B-11-50-methacrylat | H | H | -CH₃ | -Butyl | 17 | 13 |
| ®MPEG-750-methacrylat | H | H | -CH₃ | -Methyl | 18 | 0 |
| ®P-010-acrylat | H | H | H | -Phenyl | 10 | 0 |
| ®O-050-acrylat | H | H | H | -Oleyl | 5 | 0 |

Weiterhin als Makromonomere F) insbesondere geeignet sind Ester der (Meth)acrylsäure mit
(C₁₀-C₁₈)-Fettalkoholpolyglykolethern mit 8 EO-Einheiten (Genapol® C-080)
C₁₁-Oxoalkoholpolyglykolethern mit 8 EO-Einheiten (Genapol® UD-080)
(C₁₂C₁₄)-Fettalkoholpolyglykolethern mit 7 EO-Einheiten (Genapol® LA-070)
(C₁₂-C₁₄)-Fettalkoholpolyglykolethern mit 11 EO-Einheiten (Genapol® LA-110)
(C₁₆-C₁₈)-Fettalkoholpolyglykolethern mit 8 EO-Einheiten (Genapol® T-080)
(C₁₆-C₁₈)-Fettalkoholpolyglykolethern mit 15 EO-Einheiten (Genapol® T-150)
(C₁₆-C₁₈)-Fettalkoholpolyglykolethern mit 11 EO-Einheiten (Genapol® T-110)
(C₁₆-C₁₈)-Fettalkoholpolyglykolethern mit 20 EO-Einheiten (Genapol® T-200)
(C₁₆-C₁₈)-Fettalkoholpolyglykolethern mit 25 EO-Einheiten (Genapol® T-250)
(C₁₈-C₂₂)-Fettalkoholpolyglykolethern mit 25 EO-Einheiten und/oder
iso-(C₁₆-C₁₈)-Fettalkoholpolyglykolethern mit 25 EO-Einheiten.
Bei den Genapol®-Typen handelt es sich um Produkte der Firma Clariant GmbH.

Bevorzugt beträgt das Molekulargewicht der Makromonomeren F) 200 g/mol bis 10⁶ g/mol, besonders bevorzugt 150 bis 10⁴ g/mol und insbesondere bevorzugt 200 bis 5000 g/mol.

Bezogen auf die Gesamtmasse der Copolymere kann der Gehalt an Makromonomeren bis zu 99,9 Gew.-% betragen. Bevorzugt finden die Bereiche 0,5 bis 30 Gew.-% und 70 bis 99,5 Gew.-% Anwendung. Besonders bevorzugt sind Bereiche von 1 bis 20 Gew.-% und 75 bis 95 Gew.-%.

Bevorzugt als Copolymere sind solche, die durch Copolymerisation mindestens der Komponenten A), C) und D) erhältlich sind.

Weiterhin bevorzugt als Copolymere sind solche, die durch Copolymerisation mindestens der Komponenten A), C) und E) erhältlich sind.

Weiterhin bevorzugt als Copolymere sind solche, die durch Copolymerisation mindestens der Komponenten A), C) und F) erhältlich sind.

Weiterhin bevorzugt als Copolymere sind solche, die durch Copolymerisation mindestens der Komponenten A), B) und F) erhältlich sind.

Weiterhin bevorzugt als Copolymere sind solche, die durch Copolymerisation mindestens der Komponenten A), D) und F) erhältlich sind.

Weiterhin bevorzugt als Copolymere sind solche, die durch Copolymerisation mindestens der Komponenten A) und F) erhältlich sind.

Weiterhin bevorzugt als Copolymere sind solche, die durch Copolymerisation mindestens der Komponenten A) und D) erhältlich sind.

Weiterhin bevorzugt als Copolymere sind solche, die durch Copolymerisation mindestens der Komponenten A) und E) erhältlich sind.

In einer bevorzugten Ausführungsform wird die Copolymerisation in Gegenwart mindestens eines polymeren Additivs G) durchgeführt, wobei das Additiv G) vor der eigentlichen Copolymerisation dem Polymerisationsmedium ganz- oder teilweise gelöst zugegeben wird. Die Verwendung von mehreren Additiven G) ist ebenfalls erfindungsgemäß. Vernetzte Additive G) können ebenfalls verwendet werden.
Die Additive G) bzw. deren Mischungen müssen lediglich ganz oder teilweise im gewählten Polymerisationsmedium löslich sein. Während des eigentlichen Polymerisationsschrittes hat das Additiv G) mehrere Funktionen. Einerseits verhindert es im eigentlichen Polymerisationsschritt die Bildung übervemetzter Polymeranteile im sich bildenden Copolymerisat und andererseits wird das Additiv G) gemäß dem allgemein bekannten Mechanismus der Pfropfcopolymerisation statistisch von aktiven Radikalen angegriffen. Dies führt dazu, dass je nach Additiv G) mehr oder weniger große Anteile davon in die Copolymere eingebaut werden. Zudem besitzen geeignete Additive G) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive G) hergestellt wurden, zeigen solche, die unter Zusatz von Additiven G) hergestellt wurden, vorteilhafterweise eine signifikant höhere Viskosität in wässriger Lösung.
Bevorzugt als Additive G) sind in Wasser und/oder Alkoholen, bevorzugt in t-Butanol, lösliche Homo- und Copolymere. Unter Copolymeren sind dabei auch solche mit mehr als zwei verschiedenen Monomertypen zu verstehen.

Besonders bevorzugt als Additive G) sind Homo- und Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)-ethyl]trimethylammoniumchlorid (MAPTAC); Polyalkylenglykole und/oder Alkylpolyglykole.
Insbesondere bevorzugt als Additive G) sind Polyvinylpyrrolidone (z.B. Luviskol K15®, K20® und K30® von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können.

Das Molekulargewicht der Additive G) beträgt bevorzugt 10² bis 10⁷ g/mol, besonders bevorzugt 0,5*10⁴ bis 10⁶ g/mol.

Die Einsatzmenge des polymeren Additivs G) beträgt, bezogen auf die Gesamtmasse der bei der Copolymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

In einer weiteren bevorzugten Ausführungsform sind die erfindungsgemäßen Copolymere vemetzt, d.h. sie enthalten Comonomere mit mindestens zwei polymerisationsfähigen Vinylgruppen.
Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.
Insbesondere bevorzugt als Vernetzer ist Trimethylolpropantriacrylat (TMPTA). Der Gewichtsanteil an vernetzenden Comonomeren, bezogen auf die Gesamtmasse der Copolymere, beträgt bevorzugt bis 20 Gew.-%, besonders bevorzugt 0,05 bis 10 Gew.-% und insbesondere bevorzugt 0,1 bis 7 Gew.-%.

Als Polymerisationsmedium können alle organischen oder anorganischen Lösungsmittel dienen, die sich bezüglich radikalischer Polymerisationsreaktionen. weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid oder Azoinitiatoren, wie z.B. Azodiisobutyronitril (AIBN), verwendet werden. Ebenfalls geeignet sind anorganische Peroxyverbindungen, wie z.B. (NH₄)₂S₂O₈, K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.

Als Polymerisationsmedium können alle Lösungsmittel dienen, die sich bezüglich radikalischer Polymerisationsreaktionen weitestgehend inert verhalten und die Bildung hoher Molekulargewichte zulassen. Bevorzugt Verwendung finden Wasser und niedere, tertiäre Alkohole oder Kohlenwasserstoffe mit 3 bis 30 C-Atomen. In einer besonders bevorzugten Ausführungsweise wird t-Butanol als Reaktionsmedium verwendet. Mischungen aus zwei- oder mehreren Vertretern der beschriebenen potentiellen Lösungsmitteln sind selbstverständlich ebenfalls erfindungsgemäß. Dies schließt auch Emulsionen von nicht miteinander
mischbaren Solventien ein (z.B. Wasser/Kohlenwasserstoffe). Grundsätzlich sind alle Arten der Reaktionsführung geeignet, die zu den erfindungsgemäßen Polymerstrukturen führen (Lösungspolymerisation, Emulsionsverfahren, Fällungsverfahren, Hochdruckverfahren, Suspensionsverfahren, Substanzpolymerisation, Gelpolymerisation usw.).
Bevorzugt eignet sich die Fällungspolymerisation, besonders bevorzugt die Fällungspolymerisation in tert.-Butanol.

Die nachfolgende Auflistung zeigt 67 Copolymere, die für die Formulierung der erfindungsgemäßen Mittel besonders vorteilhaft geeignet sind. Die verschiedenen Copolymere Nr. 1 bis Nr. 67 sind gemäß den folgenden Herstellverfahren 1, 2, 3 und 4 erhältlich.

### Verfahren 1:

Diese Polymere sind nach dem Fällungsverfahren in tert. Butanol herstellbar. Dabei wurden die Monomere in t-Butanol vorgelegt, die Reaktionsmischung inertisiert und anschließend die Reaktion nach Anheizen auf 60°C durch Zugabe des entsprechenden t-Butanol löslichen Initiators (bevorzugt Dilauroylperoxid) gestartet. Die Polymere werden nach beendeter Reaktion (2 Stunden) durch Absaugen des Lösungsmittels und durch anschließende Vakuumtrocknung isoliert.

### Verfahren 2:

Diese Polymere sind nach dem Gelpolymerisationsverfahren in Wasser herstellbar. Dabei werden die Monomere in Wasser gelöst, die Reaktionsmischung inertisiert und anschließend die Reaktion nach Anheizen auf 65°C durch Zugabe von geeigneten Initiatoren- oder Initiatorsystemen (bevorzugt Na₂S₂O₈) gestartet. Die Polymergele werden anschließend zerkleinert und nach Trocknung die Polymere isoliert.

### Verfahren 3:

Diese Polymere sind nach dem Emulsionsverfahren in Wasser herstellbar. Dabei werden die Monomere in einer Mischung aus Wasser/organ. Lösungsmittel (bevorzugt Cyclohexan) unter Verwendung eines Emulgators emulgiert, die Reaktionsmischung mittels N₂ inertisiert und anschließend die Reaktion nach Anheizen auf 80°C durch Zugabe von geeigneten Initiatoren- oder Initiatorsystemen (bevorzugt Na₂S₂O₈) gestartet. Die Polymeremulsionen werden anschließend eingedampft (Cyclohexan fungiert als Schlepper für Wasser) und dadurch die Polymere isoliert.

### Verfahren 4:

Diese Polymere sind nach dem Lösungsverfahren in organischen Lösungsmitteln (bevorzugt Toluol, z.B. auch tert. Alkohole) herstellbar. Dabei werden die Monomere im Lösungsmittel vorgelegt, die Reaktionsmischung inertisiert und anschließend die Reaktion nach Anheizen auf 70°C durch Zugabe von geeigneten Initiatoren- oder Initiatorsystemen (bevorzugt Dilauroylperoxid) gestartet. Die Polymere werden durch Abdampfen des Lösungsmittels und durch anschließende Vakuumtrocknung isoliert.

Polymere mit hydrophoben Seitenketten, unvernetzt

| Nr. | Zusammensetzung | Herstellverfahren |
|---|---|---|
| 1 | 95 g AMPS 5 g Genapol T-080 | 1 |
| 2 | 90 g AMPS 10 g Genapol T-080 | 1 |
| 3 | 85 g AMPS 15 g Genapol T-080 | 1 |
| 4 | 80 g AMPS 20 g Genapol T-080 | 1 |
| 5 | 70 g AMPS 30 g Genapol T-080 | 1 |
| 6 | 50 g AMPS 50 g Genapol T-080 | 3 |
| 7 | 40 g AMPS 60 g Genapol T-080 | 3 |
| 8 | 30 g AMPS 70 g Genapol T-080 | 3 |
| 9 | 20 g AMPS 80 g Genapol T-080 | 3 |
| 10 | 60 g AMPS 60 g BB10 | 4 |
| 11 | 80 g AMPS 20 g BB10 | 4 |
| 12 | 90 g AMPS 10 g BB10 | 3 |
| 13 | 80 g AMPS 20 g BB10 | 1 |
| 14 | 80 g AMPS 20 g Genapol LA040 | 1 |

Polymere mit hydrophoben Seitenketten, vernetzt

| Nr. | Zusammensetzung | Herstellverfahren |
|---|---|---|
| 15 | 80 g AMPS 20 g Genapol LA040 0.6g AMA | 1 |
| 16 | 80 g AMPS 20 g Genapol LA040 0,8 AMA | 1 |
| 17 | 80 g AMPS 20 g Genapol LA040 1,0 g AMA | 1 |
| 18 | 628,73 g AMPS 120,45 g Genapol T-250 6,5 g TMPTA | 2 |
| 19 | 60 g AMPS 40 g BB10 1,9 g TMPTA | 4 |
| 20 | 80 g AMPS 20 g BB10 1,4 g TMPTA | 4 |
| 21 | 90 g AMPS 10 g BB10 1,9 g TMPTA | 4 |
| 22 | 80 g AMPS 20 g BB10 1,9 g TMPTA | 4 |
| 23 | 60 g AMPS 40 g BB10 1,4 g TMPTA | 4 |

Polymere mit hydrophoben Seitenketten, vernetzt, gepfropft

| Nr. | Zusammensetzung | Herstellverfahren |
|---|---|---|
| 24 | 95 g AMPS 5 g BB10, 1,9 g TMPTA, 1 g Poly-NVP | 1 |
| 25 | 90 g AMPS 10 g BB10, 1,9 g TMPTA, 1 g Poly-NVP | 1 |
| 26 | 85 g AMPS 15 g BB10, 1,9 g TMPTA, 1 g Poly-NVP | 1 |
| 27 | 90 g AMPS 10 g BB10, 1,9 g TMPTA, 1 g Poly-NVP | 1 |

Polymere mit siliziumhaltigen Gruppen, unvernetzt

| Nr. | Zusammensetzung | Herstellverfahren |
|---|---|---|
| 28 | 80 g AMPS, 20 g Silvet 867 | 1 |
| 29 | 80 g AMPS, 50 g Silvet 867 | 4 |

Polymere mit siliziumhaltigen Gruppen, vernetzt

| Nr. | Zusammensetzung | Herstellverfahren |
|---|---|---|
| 30 | 80 g AMPS, 20 g Silvet 867, 0,5 g MBA | 4 |
| 31 | 80 g AMPS, 20 g Silvet 867, 1,0 g MBA | 1 |
| 32 | 60 g AMPS, 40 g Y-12867, 0,95 g AMA | 1 |
| 33 | 80 g AMPS, 20 g Y-12867, 0,95 g AMA | 1 |
| 34 | 90 g AMPS, 10 g Y-12867, 0,95 g AMA | 1 |
| 35 | 60 g AMPS, 40 g Silvet 7280, 0,95 g AMA | 1 |
| 36 | 80 g AMPS, 20 g Silvet 7280, 0,95 g AMA | 1 |
| 37 | 90 g AMPS, 10 g Silvet 7280, 0,95 g AMA | 1 |
| 38 | 60 g AMPS, 40 g Silvet 7608, 0,95 g AMA | 1 |
| 39 | 80 g AMPS, 20 g Silvet 7608, 0,95 g AMA | 1 |
| 40 | 90 g AMPS, 10 g Silvet 7608, 0,95 g AMA | 1 |

Polymere mit hydrophoben Seitenketten und kationischen Gruppen, unvernetzt

| Nr. | Zusammensetzung | Herstellverfahren |
|---|---|---|
| 41 | 87,5 g AMPS, 7,5 g Genapol T-110, 5 g DADMAC | 2 |
| 42 | 40 g AMPS, 10 g Genapol T110, 45 g Methacrylamid | 2 |
| 43 | 55 g AMPS, 40 g Genapol LA040, 5 g Quat | 1 |
| 44 | 75 g AMPS, 10 g BB10, 6,7 g Quat | 1 |

Polymere mit hydrophoben Seitenketten und kationischen Gruppen, vernetzt

| Nr. | Zusammensetzung | Herstellverfahren |
|---|---|---|
| 45 | 60 g AMPS, 20 g Genapol T-80, 10 g Quat, 10 g HEMA | 1 |
| 46 | 75 g AMPS, 20 g GenapolT-250, 5 g Quat, 1,4 g TMPTA | 1 |
| 47 | 75 g AMPS, 20 g GenapolT-250, 10 g Quat, 1,4 g TMPTA | 1 |
| 48 | 75 g AMPS, 20 g GenapolT-250, 20 g Quat, 1,4 g TMPTA | 1 |

Polymere mit fluorhaltigen Gruppen

| Nr. | Zusammensetzung | Herstellverfahren |
|---|---|---|
| 49 | 94 g AMPS, 2,02 g Fluowet AC 600 | 1 |
| 50 | 80 g AMPS, 20 g Perfluoroctylpolyethylenglykolmethacrylat, 1 g Span 80 | 3 |

Polymere mit fluorhaltigen Gruppen, gepfropft

| Nr. | Zusammensetzung | Herstellverfahren |
|---|---|---|
| 51 | 80 g AMPS, 10 g Fluowet AC 600, 5 g Poly-NVP | 1 |
| 52 | 70 g AMPS, 8 g Perfluoroctylethyloxyglycerinmethacrylat, 5 g Poly-NVP | 4 |

Multifunktionelle Polymere

| Nr. | Zusammensetzung | Herstellverfahren |
|---|---|---|
| 53 | 80 g AMPS, 10 g Genapol LA070, 10 g Silvet 7608, 1,8 g TMPTA | 1 |
| 54 | 70 g AMPS, 5 g N-Vinylpyrrolidon, 15 g Genapol T-250 methacrylat, 10 g Quat, 10 g Poly-NVP | 4 |
| 55 | 80 g AMPS, 5 g N-Vinylformamid, 5 g Genapol O-150-methacrylat, 10 g DADMAC, 1,8 g TMPTA, 8 g Poly-N-Vinylformamid | 2 |
| 56 | 70 g AMPS, 5 g N-Vinylpyrrolidon, 15 g Genapol T-250-methacrylat, 10 g Quat, 10 g Poly-NVP | 1 |
| 57 | 60 g AMPS, 10 g Genapol-BE-020-methacrylat, 10 g Genapol T-250-acrylat, 20 g Quat, 1 g Span 80 | 1 |
| 58 | 60 g AMPS, 20 g MPEG-750-methacrylat, 10 g Methacryloxypyldimethicon, 10 g Perfluorooctylpolyethylenglycol-methacrylat, 10 g Poly[N-vinylcaprolacton-co-acrylsäure] (10/90) | 1 |
| 59 | 80 g AMPS, 5 g N-Vinylformamid, 5 g Genapol O-150-methacrylat, 10 g DADMAC, 1,8 g TMPTA | 1 |
| 60 | 70 g AMPS, 10 g Genapol T-250-acrylat, 5 g N-Methyl-4-vinylpyridiniumchlorid, 2,5 g Silvet Y-12867, 2,5 g Perfluorhexylpolyethylenglykolmethacrylat, 10 g Polyethylenglykoldimethacrylat, 4 g Poly[N-Vinylcaprolactam] | 1 |
| 61 | 10 g AMPS, 20 g Acrylamid, 30 g N-2-Vinylpyrrolidon, 20 g Silvet 7608, 10 g Methacryloxypyl dimethicon, 10 g Fluowet AC 812 | 3 |
| 62 | 60 g AMPS, 10 g DADMAC, 10 g Quat, 10 g Genapol-LA-250-crotonat, 10 g Methacryloxypyldimethicon, 7 g Poly[acrylsäure-co-N-vinylformamid] | 1 |
| 63 | 50 g AMPS, 45 g Silvet 7608, 1,8 g TMPTA, 8 g Poly[N-Vinylformamid] | 1 |
| 64 | 20 g AMPS, 10 g Genapol T 110, 35 g MAA, 30 g HEMA, 5 g DADMAC | 4 |
| 65 | 20 g AMPS, 80gBB10, 1,4 g TMPTA | 1 |
| 66 | 75 g AMPS, 20gBB10, 6,7 g Quat, 1,4 g TMPTA | 1 |
| 67 | 35 g AMPS, 60 g Acrylamid, 2 g VIFA, 2,5 g Vinylphosphonsäure, 2 Mol-% Fluowet EA-600 | 4 |

Chemische Bezeichnung der Reaktanden:
- AMPS: Acryloyldimethyltaurat, bevorzugt Na- oder NH4-Salz
- Genapol® T-080: C₁₆-C₁₈-Fettalkoholpolyglykolether mit 8 EO-Einheiten
- Genapol® T-110: C₁₂-C₁₄-Fettalkoholpolyglykolether mit 11 EO-Einheiten
- Genapol® T-250: C₁₆-C₁₈-Fettalkoholpolyglycolether mit 25 EO-Einheiten
- Genapol® LA-040: C₁₂-C₁₄-Fettalkoholpolyglykolether mit 4 EO-Einheiten
- Genapol® LA-070: C₁₂-C₁₄-Fettalkoholpolyglykolether mit 7 EO-Einheiten
- Genapol® O-150 methacrylat: C₁₆-C₁₈-Fettalkoholpolyglykolether methacrylat mit 15 EO-Einheiten,
- Genapol® LA-250 crotonat: C₁₂-C₁₄-Fettalkoholpolyglykolether crotonat mit 25 EO-Einheiten
- Genapol® T-250 methacrylat: C₁₆-C₁₈-Fettalkoholpolyglycolether methacrylat mit 25 EO-Einheiten
- Genapol® T-250 acrylat: C₁₆-C₁₈-Fettalkoholpolyglycolether methacrylat mit 25 EO-Einheiten
- BB10®: Polyoxyethylen(10)Behenylether
- TMPTA: Trimethylolpropantriacrylat
- Poly-NVP: Poly-N-Vinylpyrrolidon
- Silvet® 867: Siloxan Polyalkylenoxid Copolymer
- MBA: Methylen-bis-acrylamid
- AMA: Allylmethacrylat
- ®Y-12867: Siloxan Polyalkylenoxid Copolymer
- Silvet® 7608: Polyalkylenoxid-modifiziertes Heptamethyltrisiloxan
- Silvet® 7280: Polyalkylenoxid-modifiziertes Heptamethyltrisiloxan
- DADMAC: Diallyldimethyl-ammoniumchlorid
- HEMA: 2-Hydroxyethylmethacrylat
- Quat: 2-(Methacryloyloxy)ethyltrimethylammoniumchlorid
- Fluowet® AC 600: Perfluoralkylethylacrylat
- Span® 80: Sorbitanester

Die beschriebene, optional durchführbare Pfropfung der Copolymere mit anderen Polymeren führt zu Produkten mit besonderer Polymermorphologie, die in wässrigen Systemen optisch klare Gele ergeben. Ein potenzieller Nachteil der Copolymere ohne Pfropfung besteht in einer mehr oder weniger starken Opaleszenz in wässriger Lösung. Diese beruht auf bisher nicht zu vermeidenden, übervernetzten Polymeranteilen, die während der Synthese entstehen und in Wasser nur unzureichend gequollen vorliegen. Dadurch bilden sich Licht streuende Teilchen aus, deren Größe deutlich oberhalb der Wellenlänge des sichtbaren Lichts liegt und deshalb Ursache der Opaleszenz sind. Durch das beschriebene, optional durchführbare Pfropf-Verfahren wird die Bildung übervernetzter Polymeranteile gegenüber herkömmlichen Techniken deutlich reduziert oder gänzlich vermieden.

Die beschriebene, optional durchführbare Inkorporation sowohl von kationischen Ladungen als auch von Silizium-, Fluor oder Phosphoratomen in die Copolymere führt zu Produkten, die in kosmetischen Formulierungen besondere sensorische und rheologische Eigenschaften besitzen. Eine Verbesserung der sensorischen und rheologischen Eigenschaften kann insbesondere bei der Verwendung in rinse off Produkten (insbesondere Haarbehandlungsmittel) als auch leave on Produkten (insbesondere O/W Emulsionen) gewünscht sein.

Vorteilhafte Eigenschaften zeigen die Copolymere sowohl in vernetzter als auch in unvernetzter Form. Während vemetzte Systeme z.B. hervorragende Eigenschaftsprofile im Hinblick auf Emulsionsstabilisierung zeigten, konnten insbesondere mit Hilfe der unvernetzten Varianten tensidhaltige Lösungen verdickt werden. Gleiches gilt für elektrolythaltige Systeme, die bekanntermaßen mit Polyelektrolyten nur sehr schwer oder gar nicht zu verdicken sind.

Die Copolymere können als Verdicker für Mittel auf wässriger oder wässrigalkoholischer Basis, beispielsweise Haargele, eingesetzt werden. Des weiteren eignen sich die erfindungsgemäßen Polymere als Stabilisator, Dispergiermittel und Konsistenzgeber für wässrig-tensidische Zubereitungen, beispielsweise Shampoos, Duschbäder, Duschgels, Schaumbäder und dergleichen. Die verdickende Wirkung der Copolymere in wässrig-tensidischen Mitteln wird durch eine Assoziation der Polymerseitenketten und der Tenside verstärkt und kann durch die Wahl der Seitenketten der Copolymere und durch die Wahl der Tenside gesteuert werden. Die suspendierende bzw. dispergierende und stabilisierende Wirkung der Copolymere in wässrig-tensidischen Mitteln wird durch die Assoziation der Polymerseitenketten bzw. funktionellen Gruppen in Haupt- und Seitenkette und der in wässrig-tensidischen Mitteln unlöslichen flüssigen Komponenten, beispielsweise Silikonöle, bzw. der unlöslichen Komponenten, beispielsweise Zink-Pyrethione, bedingt.

Die Copolymere eignen sich ebenso als Verdicker und Dispergiermittel, als Emulgatoren, Suspendiermittel mit verdickender Wirkung und Konsistenzgeber für Emulsionen und Suspensionen, sowie als Gleitmittel, Haftmittel, Verdicker, Dispergier- und Emulgiermittel dekorativer, feststoffhaltiger Zubereitungen. Dabei können auch Mischungen der Copolymere verwendet werden. Die emulgierende, stabilisierende und/oder konsistenzgebende Wirkung der Copolymere in Emulsionen wird durch eine Assoziation der Polymerseitenketten untereinander, sowie durch eine Wechselwirkung der Polymerseitenketten mit den hydrophoben Ölkomponenten verursacht bzw. verstärkt.

Als Elektrolyt enthalten die erfindungsgemäßen Mittel Salze. Bei den Salzen handelt-es sich um heteropolare Verbindungen, an deren Kristallgitter mindestens eine von Wasserstoff-Ionen verschiedene Kationen-Art und mindestens eine von Hydroxid-Ionen verschiedene Anionen-Art beteiligt sind. Bevorzugt enthalten die erfindungsgemäßen Mittel anorganische Salze, besonders bevorzugt Ammonium- oder Metallsalze, bevorzugt von Halogeniden, Oxiden, Carbonaten, Hydrogencarbonaten, Phosphaten, Sulfaten, Nitraten, insbesondere bevorzugt Natriumchlorid; und/oder organische Salze, bevorzugt Ammonium- oder Metallsalze, bevorzugt der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure, Galacturonsäure.

Die Salze sind bevorzugt von ein- oder mehrwertigen Säuren und Basen abgeleitet, bevorzugt von einwertigen Säuren und/oder einwertigen Basen. Besonders bevorzugt sind Natrium-, Kalium und Ammoniumsalze.

Als Elektrolyt können die Mittel auch Mischungen verschiedener Salze enthalten. Die Mittel enthalten die Elektrolyte üblicherweise in einer Konzentration von 0,01 bis 50 Gew.-%, bevorzugt 0,1 bis 50 Gew.-%, besonders bevorzugt 0, 5 bis 10 Gew.-%.

Die Mittel haben bevorzugt eine Ionenstärke I im Bereich 10⁻⁵ mol/l bis 2 mol/l, besonders bevorzugt 10⁻² mol/l bis 1 mol/l, insbesondere bevorzugt 0,1 mol/l bis 0,75 mol/l. Die Ionenstärke I ist definiert als I = 0,5 ∑ cᵢ*zᵢ², wobei cᵢ die molare Konzentration der einzelnen Ionensorten i und zᵢ die Ionenladungen der Ionensorten i darstellen.

Die Verdickungsleistung der kammförmigen Copolymere auf Basis von Acryloyldimethyltaurinsäure ist abhängig von der Salz- und Copolymerkonzentration und vom Grad der Substitution der kammförmigen Copolymere. Durch Variation von Salzmenge, Copolymermenge und Substitutionsgrad wird die Viskosität der pharmazeutischen und dermatologischen Mitteln eingestellt.

Bei den Mitteln handelt es sich bevorzugt um Emulsionen, besonders bevorzugt um Öl-in-Wasser-Emulsionen mit Viskositäten von 8000 mPas bis 50000 mPas (RV Brookfield Viskosimeter, 20 U/min), wässrige Gele, besonders bevorzugt um wässrige Gele enthaltend organische Lösemittel mit Viskositäten von 15000 mPas bis 100000 mPas mPas (RV Brookfield Viskosimeter, 20 U/min), tensidhaltige Formulierungen, besonders bevorzugt um Shampoos, Duschbäder und dergleichen mit Viskositäten von 1000 mPas bis 15000 mPas mPas (RV Brookfield Viskosimeter, 20 U/min).

Die erfindungsgemäßen Mittel enthalten bezogen auf die fertigen Mittel, bevorzugt 0,01 bis 10 Gew.-%, besonders bevorzugt 0,1 bis 5 Gew.-%, insbesondere bevorzugt 0,5 bis 3 Gew.-%, an Copolymeren.

Die erfindungsgemäßen Mittel sind üblicherweise auf einen pH Wert im Bereich 2 bis 12, bevorzugt pH 3 bis 8, eingestellt.

Die erfindungsgemäßen Mittel können anionische, kationische, nichtionische, zwitterionische und/oder amphotere Tenside enthalten.

Die Gesamtmenge der eingesetzten Tenside beträgt, bezogen auf die fertigen Mittel, bevorzugt zwischen 2 bis 70 Gew.-%, besonders bevorzugt zwischen 5 und 40 Gew.-%, insbesondere bevorzugt zwischen 12 und 35 Gew.-%.

Als anionische Tenside eignen sich bevorzugt (C₁₀-C₂₀)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäure-. methyltauride, Fettsäuresarkosinate, Sulforicinoleate, Acylglutamate.
Die Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside liegt, bezogen auf die fertigen Mittel, bevorzugt im Bereich von 2 bis 30 Gew.-%, besonders bevorzugt 5 bis 25 Gew.-%, insbesondere bevorzugt 12 bis 22 Gew.-%.

Geeignete kationische Tenside sind beispielsweise quartäre Ammoniumsalze wie Di-(C₁₀-C₂₄)-Alkyl-dimethylammoniumchlorid oder -bromid, vorzugsweise Di-(C₁₂-C₁₈)-Alkyl-dimethylammoniumchlorid oder -bromid; (C₁₀-C₂₄)-Alkyl-dimethylethylammoniumchlorid oder -bromid; (C₁₀-C₂₄)-Alkyl-trimethylammoniumchlorid oder -bromid, vorzugsweise Cetyltrimethylammoniumchlorid oder -bromid und (C₂₀-C₂₂)-Alkyl-trimethylammoniumchlorid oder -bromid; (C₁₀-C₂₄)-Alkyl-dimethylbenzylammoniumchlorid oder -bromid, vorzugsweise (C₁₂-C₁₈)-Alkyl-dimethylbenzylammoniumchlorid; N-(C₁₀-C₁₈)-Alkyl-pyridiniumchlorid oder -bromid, vorzugsweise N-(C₁₂-C₁₆)-Alkyl-pyridiniumchlorid oder -bromid; N-(C₁₀-C₁₈)-Alkyl-isochinoliniumchlorid, -bromid oder -monoalkylsulfat; N-(C₁₂-C₁₈)-Alkyl-polyoylaminoformylmethylpyridiniumchlorid; N-(C₁₂-C₁₈)-Alkyl-N-methyl-morpholinium-chlorid, -bromid oder -monoalkylsulfat; N-(C₁₂-C₁₈)-Alkyl-N-ethyl-morpholinium-chlorid, -bromid oder -monoalkylsulfat; (C₁₆-C₁₈)-Alkyl-pentaoxethyl-ammonium-chlorid; Diisobutylphenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside beträgt, bezogen auf die fertigen Mittel, bevorzugt 1 bis 10 Gew.-%, besonders bevorzugt 2 bis 7 Gew.-%, insbesondere besonders bevorzugt 3 bis 5 Gew.-%.

Als nichtionische Tenside eignen sich Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics®); Fettsäurealkylolamide, (Fettsäureamidpolyethylenglykole); N-Alkyl-, N-Alkoxypolyhydroxyfettsäureamid, Saccharoseester; Sorbitester und Polyglykolether.

Der Gewichtsanteil der nichtionischen Tenside beträgt bevorzugt 1 bis 20 Gew.-%, besonders bevorzugt 2 bis 10 %, insbesondere bevorzugt 3 bis 7 Gew.-%.

Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze; N-Acylaminoalkyl-N,N-dimethyl-acetobetain, vorzugsweise N-(C₈-C₁₈)-Acylaminopropyl-N,N-dimethylacetobetain; (C₁₂-C₁₈)-Alkyl-dimethyl-sulfopropyl-betain; Amphotenside auf Basis Imidazolin (Handelsname: Miranol®, Steinapon®), vorzugsweise das Natrium-Salz des 1-(β-Carboxy-methyloxyethyl)-1-(carboxymethyl)-2-lauryl-imidazoliniums; Aminoxid, z.B. (C₁₂-C₁₈)-Alkyldimethylaminoxid, Fettsäureamidoalkyl-dimethylaminoxid.

Der Gewichtsanteil der amphoteren Tenside liegt bevorzugt im Bereich von 0,5 bis 20 Gew.-%, besonders bevorzugt 1 bis 10 Gew.-%.

Besonders bevorzugte Tenside sind Laurylsulfat, Laurethsulfat, Cocoamidopropylbetain, Natriumcocoylglutamat und Lauroamphoacetat.

Des weiteren können in den erfindungsgemäßen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Die erfindungsgemäßen Mittel können als weitere Hilfs- und Zusatzstoffe Ölkörper, Emulgatoren und Co-Emulgatoren, kationische Polymere, Filmbildner, sowie weitere in der Kosmetik gebräuchliche Zusätze, wie z.B. Überfettungsmittel, feuchtigkeitsspendende Mittel, Stabilisatoren, biogene Wirkstoffe, Glycerin, Konservierungsmittel, Perlglanzmittel, Farb- und Duftstoffe, Lösungsmittel, Hydrotrope, Trübungsmittel, weitere Verdickungsmittel und Dispergiermittel ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen, Antioxidation, UV-Lichtschutzfilter, Pigmente und Metalloxide, sowie antimikrobiell wirkende Agentien enthalten.

Unter Ölkörper ist jegliche Fettsubstanz zu verstehen, die bei Raumtemperatur (25°C) flüssig ist.
Die Fett-Phase kann daher ein oder mehrere Öle umfassen, die vorzugsweise aus folgenden Ölen ausgewählt werden:
Silikonöle, flüchtig oder nicht flüchtig, linear, verzweigt oder ringförmig, eventuell organisch modifiziert; Phenylsilikone; Silikonharze und -gummis; Mineralöle wie Paraffin- oder Vaselinöl; Öle tierischen Ursprungs wie Perhydrosqualen, Lanolin; Öle pflanzlichen Ursprungs wie flüssige Triglyceride, z.B. Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Triglyceride der Capryl/Caprinsäuren, Olivenöl, Erdnussöl, Rapsöl und Kokosnussöl;
Synthetische Öle wie Purcellinöl, Isoparaffine, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren
mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Triglyceride auf Basis (C₆-C₁₀)-Fettsäuren;
Ester wie Dioctyladipate, Diisopropyl dimer dilineloate; Propylenglykole/-dicaprilate oder Wachse wie Bienenwachs, Paraffinwachs oder Mikrowachse, gegebenenfalls in Kombination mit hydrophilen Wachsen, wie z.B. Cetylstearylalkohol; Fluorierte und perfluorierte Öle; fluorierte Silikonöle; Gemische der vorgenannten Verbindungen.
Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, di- und tri-Alkylquats und deren polymere Derivate.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guar-hydroxypropyltriammoniumchloride, sowie Calciumalginat und Ammoniumalginat. Des weiteren können eingesetzt werden kationische Cellulosederivate; kationische Stärke; Copolymere von Diallylammoniumsalzen und Acrylamiden; quaternierte VinylpyrrolidonNinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan. Geeignete Siliconverbindungen sind beispielsweise Dimethylpolysiloxan, Methylphenylpolysiloxane, cyclische Silicone und amino-, fettsäure-, alkohol-, polyether-, epoyx-, fluor- und/oder alkylmodifizierte Siliconverbindungen, sowie Polyalkylsiloxane, Polyalkylarylsiloxane, Polyethersiloxan-Copolymere, wie in US 5 104 645 und den darin zitierten Schriften beschrieben, die bei Raumtemperatur sowohl flüssig als auch harzförmig vorliegen können.

Geeignete Filmbildner sind, je nach Anwendungszweck Salze der Phenylbenzimidazolsulfonsäure, wasserlösliche Polyurethane, beispielsweise C₁₀-Polycarbamylpolyglycerylester, Polyvinylalkohol, Polyvinylpyrrolidon, -copolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carbocyvinyl-Polymere, wie Carbomere und deren Salze, Polysaccharide, beispielsweise Polydextrose und Glucan.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als feuchtigkeitsspendende Substanz stehen beispielsweise Isopropylpalmitat, Glycerin und/oder Sorbitol zu Verfügung.

Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen.

Zusätzlich können die erfindungsgemäßen Mittel organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol. Hydrotrop wirken kurzkettige Aniontenside, insbesondere Arylsulfonate, beispielsweise Cumol- oder Toluolsulfonat.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Als Konservierungsmittel in Betracht kommen beispielsweise Phenoxyethanol, Parabene, Pentandiol oder Sorbinsäure.

Als Farbstoffe können die für kosmetische Zwecke geeigneten und zugelassenen Substanzen verwendet werden.

Als antifungizide Wirkstoffe eignen sich bevorzugt Ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Oczopyrox.

Die erfindungsgemäßen Mittel sind üblicherweise auf einen pH Wert im Bereich 2 bis 12, bevorzugt pH 3 bis 8, eingestellt.

In einer bevorzugten Ausführungsform handelt es sich bei den Mitteln um Rinse-off Produkte, besonders bevorzugt Shampoos, Duschbäder, Duschgels und Schaumbäder. Moderne Rinse-off Produkte haben häufig einen hohen Anteil an konditionierenden Wirkstoffen, die auch aus Ölanteilen bestehen können. Folglich können diese Mittel als Emulsionen vorliegen.

In einer weiteren bevorzugten Ausführungsform handelt es sich bei den Mitteln um Leave-on Produkte, bevorzugt in Form von Emulsionen, besonders bevorzugt um Hautpflegemittel Tagescremes, Nachtcremes, Pflegecremes, Nährcremes, Bodylotions, Salben, Sonnenschutzmittel, Lippenpflegemittel und Deodorantien.

Des weiteren eignen sie sich auch für tensidfreie wässrige Mittel und Emulsionen, beispielsweise für Haarkuren und -spülungen, Haargele aber auch für Dauerwellenmittel, Haarfärbemittel, sowie für dekorative Kosmetika, beispielsweise make-ups, eye-shadows, Lippenstifte, Mascara und dergleichen.
Ein wesentlich Punkt der Erfindung ist, dass die erfindungsgemäßen Mittel auch ohne Mitverwendung eines zusätzlichen Co-Emulgators und/oder ohne Mitverwendung eines zusätzlichen Konsistenzgebers eingesetzt werden können.

Die Mitverwendung von Co-Emulgatoren und/oder Konsistenzgebern ist somit nicht zwingend, jedoch natürlich möglich. Eine Kombination mit anderen bekannten Co-Emulgatoren und/oder Konsistenzgebern kann zur Einstellung spezieller kosmetischer Profile und zur Ausnutzung synergistischer Effekte wünschenswert sein. Die nachfolgenden Beispiele dienen der Erläuterung der Erfindung, ohne sie jedoch einzuschränken (bei allen Prozentangaben handelt es sich um Gew.-%). Bei den in den Beispielen verwendeten Copolymeren handelt es sich um Vertreter der in der Beschreibung bereits aufgeführten besonders bevorzugten Copolymere Nr.1 bis Nr.67. Die Herstellung der Copolymere erfolgte nach den dort angegebenen Verfahren 1, 2, 3 oder 4 unter Verwendung der bevorzugten Initiatoren und Lösemittel.

Verdickerleistung in Abhängigkeit der Elektrolytkonzentration

**Tabelle 1:**

| Verdickungsleistung von Polymer in wässriger Lösung in Abhängigkeit der Elektrolytkonzentration | | | | | | | | |
|---|---|---|---|---|---|---|---|---|
| [Gew.-% NaCl] | 0,0 % | 1,0 % | 2,0 % | 3,0 % | 4,0 % | 5,0 % | 6,0 % | 7,0 % |
| Polymer I | 914 | 506 | 454 | 402 | 336 | 310 | 282 | 276 |
| Spindel | 2 | 2 | 2 | 2 | 2 | 2 | 2 | 2 |
| Polymer II | 2260 | 1620 | 1725 | 2010 | 2200 | 2555 | 2845 | 3370 |
| Spindel | 3 | 3 | 3 | 3 | 3 | 3 | 3 | 3 |
| Polymer III | 8050 | 11100 | 16900 | 21600 | 32600 | 38400 | 47000 | 62200 |
| Spindel | 4 | 5 | 6 | 7 | 7 | 7 | 7 | 7 |

Einsatzkonzentration Polymer in Wasser: 10 Gew.-%

| | |
|---|---|
| Polymer I: | 100 g AMPS + 0 g Genapol LA 070 |
| Polymer II: | 90 g AMPS + 10 g Genapol LA 070 |
| Polymer III: | 80 g AMPS + 20 g Genapol LA 070 |

Diese Polymere sind nach dem Fällungsverfahren in tert. Butanol (Verfahren 1) hergestellt und wurden in Form des Ammoniumsalzes eingesetzt.
Die Viskosität wurde mit einem RV-Brookfield Viskosimeter (Spindel It. Tabelle), 20 U/min, 20°C gemessen.

Das unsubstituierte Vergleichspolymer I zeigt in wässriger Lösung keine Viskosität. Die erfindungsgemäßen Polymere II und III verdicken signifikant wässrige Systeme. Die Verdickungsleistung ist abhängig von der Salzkonzentration und vom Grad der Substitution. Man erhält transparente Gele.

### Formulierungsbeispiele

### Beispiel 1: O/W-Hautmilch mit Thermalwasser

### Zusammensetzung:

| | | |
|---|---|---|
| A | Copolymer Nr. 21 | 1,50 % |
| B | Isopropylpalmitat | 4,00 % |
| | Mandelöl | 4,00 % |
| | Weizenkeimöl | 1,00 % |
| | ®Cetiol SN (Henkel) | 8,00 % |
| | Cetearylisononanoat | |
| C | Thermal-Wasser | ad 100 % |
| D | Duftstoffe | 0,30 % |

### Herstellung

| | |
|---|---|
| I | A in B unter Rühren dispergieren |
| II | C und D nacheinander zu 1 hinzufügen |
| III | Emulsion homogenisieren |

### Beispiele für Tensidformulierungen

### Beispiel 2: Duschbad mit Totes Meer Salz

### Zusammensetzung

| | | |
|---|---|---|
| A | ®GENAPOL LRO flüssig (Clariant) | 40,00 % |
| | Laurethsulfat, Na-Salz | |
| B | Duftstoff | 0,30 % |
| C | Wasser | 52,70 % |
| | Natrium Chlorid | 10,0 % |
| D | Farbstoff | q.s. |
| | Konservierungsmittel | q.s. |
| | ®GENAGEN LDA (Clariant) | 6,00 % |
| | Lauroamphodiacetat, Na-Salz | |
| | Zitronensäure | q.s. |
| E | Copolymer Nr. 1 | 1,00 % |

### Herstellung

| | |
|---|---|
| I | B in A einrühren |
| II | Die Komponenten von C mischen |
| III | Komponenten aus D nacheinander zu II zugeben |
| IV | Einstellen der Viskosität durch Einrühren von E in II |

## Patentansprüche

1. Elektrolythaltige kosmetische, dermatologische und pharmazeutische Mittel, **dadurch gekennzeichnet, dass** sie mindestens ein Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt, und wobei mindestens ein Makromonomer ein Makromonomer gemäss Formel (IV) 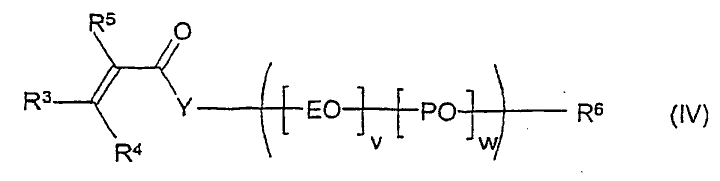 ist, worin
R₃, R₄, R₅ und R₆ unabhängig voneinander Wasserstoff oder n-aliphatische, iso-aliphatische, olefinische, cycloaliphatische, arylaliphatische oder aromatische (C₁-C₃₀)-Kohlenwasserstoffreste bedeuten,
v und w unabhängig voneinander 0 bis 500 sind,
die Summe aus v und w im Mittel ≥1 ist, und
Y eine Brücke ausgewählt aus -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)- bedeutet,
G) wobei die Copolymerisation gegebenenfalls in Gegenwart mindestens eines polymeren Additivs mit zahlenmittleren Molekulargewichten von 200 g/mol bis 10⁹ g/mol erfolgt,
H) mit der Maßgabe, dass die Komponente A) mit mindestens einer Komponente ausgewählt aus einer der Gruppen D) bis G) copolymerisiert wird,
enthalten und wobei der Gehalt an Acryloyldimethyltaurinsäure bzw. Acryloyldimethyltauraten bezogen auf die Gesamtmasse der Copolymere mindestens 0,1 Gew.-% beträgt.

2. Mittel nach Anspruch 1, **dadurch gekennzeichnet, dass** es sich bei den Comonomeren B) um ungesättigte Carbonsäuren, Salze ungesättigter Carbonsäuren, Anhydride ungesättigter Carbonsäuren, Ester ungesättigter Carbonsäuren mit aliphatischen, olefinischen, cycloaliphatischen, arylaliphatischen oder aromatischen Alkoholen mit 1 bis 22 C-Atomen, offenkettige N-Vinylamide, cyclische N-Vinylamide mit einer Ringgröße von 3 bis 9, Amide der Acrylsäure, Amide der Methacrylsäure, Amide substituierter Acrylsäuren, Amide substituierter Methacrylsäuren, 2-Vinylpyridin, 4-Vinylpyridin, Vinylacetat; Styrol, Acrylnitril, Vinylchlorid, Vinylidenchlorid, Tetrafluorethylen, Vinylphosphonsäure oder deren Ester oder Salze, Vinylsulfonsäure oder deren Ester oder Salze, Allylphosphonsäure oder deren Ester oder Salze und/oder Methallylsulfonsäure oder deren Ester oder Salze handelt.

3. Mittel nach Anspruch 1 und/oder 2, **dadurch gekennzeichnet, dass** es sich bei den Comonomeren C) um
Diallyldimethylammoniumchlorid (DADMAC),
[2-(Methacryloyloxy)ethyl]trimethylammoniumchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchlorid,
N-Methyl-4-vinylpyridiniumchlorid,
Dimethylaminoethylmethacrylat,
Dimethylaminopropylmethacrylamid,
Methacryloylethyl-N-oxid und/oder
Methacryloylethyl-betain handelt.

4. Mittel nach mindestens einem der Ansprüche 1 bis 3, **dadurch gekennzeichnet, dass** es sich bei den siliziumhaltigen Komponenten D) um Verbindungen der Formel (I)
R¹-Z-[(Si(R³R⁴)-O-)_{w}-(Si(R⁵R⁶)-O)ₓ-]-R² (I)
handelt, wobei
R¹ einen Vinyl-, Allyl-, Methallyl-, Methylvinyl-, Acryl-, Methacryl-, Crotonyl-, Senecionyl-, Itaconyl-, Maleinyl-, Fumaryl- oder ein Styrylrest darstellt;
Z eine chemische Brücke, bevorzugt ausgewählt aus -O-, -((C₁-C₅₀)Alkylen)-, -((C₆-C₃₀)Arylen)-, -((C₅-C₈)Cycloalkylen)-, -((C₁-C₅₀)Alkenylen)-, -(Polypropylenoxid)ₙ-, -(Polyethylenoxid)ₒ-, (Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann, ((C₁-C₁₀)Alkyl)-(Si(OCH₃)₂)- und -(Si(OCH₃)₂)-, darstellt;
R³, R⁴, R⁵ und R⁶ unabhängig voneinander -CH₃, -O-CH₃, -C₆H₅ oder -O-C₆H₅ bedeuten;
w, x Zahlen von 0 bis 500 bedeuten, wobei entweder w oder x größer Null sein muss, und
R² einen gesättigten oder ungesättigten, aliphatischen cycloaliphatischen, arylaliphatischen oder aromatischen Rest mit jeweils 1 bis 50 C-Atomen oder eine Gruppe der Formeln -OH, -NH₂, -N(CH₃)₂, -R⁷ oder eine Gruppe -Z-R¹ bedeutet, wobei Z und R¹ die obengenannten Bedeutungen haben und R⁷ eine Gruppe der Formel -O-Si(CH₃)₃, -O-Si(Phenyl)₃, -O-Si(O-Si(CH₃)₃)₂CH₃) und -O-Si(O-Si(Ph)₃)₂Ph) bedeutet.

5. Mittel nach mindestens einem der Ansprüche 1 bis 4, **dadurch gekennzeichnet, dass** es sich bei den fluorhaltigen Komponenten E) um Verbindungen der Formel (II)
R¹-Y-CᵣH₂ᵣCₛF₂ₛCF₃ (II)
handelt, wobei
R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine chemische Brücke, bevorzugt -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten, darstellt und
r, s stöchiometrische Koeffizienten darstellen, die unabhängig voneinander Zahlen zwischen 0 und 200 sind.

6. Mittel nach mindestens einem der Ansprüche 1 bis 5, **dadurch gekennzeichnet, dass** Komponente F) neben den Makromonomeren der Formel (IV) zusätzlich ein oder mehrere Makromonomere gemäss Formel (III) enthält,
R¹-Y-[(A)ᵥ-(B)_{w}-(C)ₓ-(D)_{z}]-R² (III)
wobei R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine verbrückende Gruppe, bevorzugt -O-, -S-, -C(O)-, -C(O)-O-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)- darstellt;
A, B, C und D unabhängig voneinander diskrete chemische Wiederholungseinheiten, bevorzugt hervorgegangen aus Acrylamid, Methacrylamid, Ethylenoxid, Propylenoxid, AMPS, Acrylsäure, Methacrylsäure, Methylmetacrylat, Acrylnitril, Maleinsäure, Vinylacetat, Styrol, 1,3-Butadien, Isopren, Isobuten, Diethylacrylamid und Diisopropylacrylamid, insbesondere bevorzugt Ethylenoxid, Propylenoxid darstellen;
v, w, x und z unabhängig voneinander 0 bis 500, bevorzugt 1 bis 30, betragen, wobei die Summe aus v, w, x und z im Mittel ≥ 1 ist; und
R² einen linearen oder verzweigten aliphatischen, olefinischen, cycloaliphatischen, arylaliphatischen oder aromatischen (C₁-C₅₀)-Kohlenwasserstoffrest, OH, -NH₂ oder -N(CH₃)₂ darstellt oder gleich [-Y-R¹] ist.

7. Mittel nach mindestens einem der Ansprüche 1 bis 6, **dadurch gekennzeichnet, dass** es sich bei den polymeren Additiven G) um Homo- oder Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxymethylmethacrylat, Diallyldimethylammoniumchlorid (DADMAC) und/oder [2-(Methacryloyloxy)ethyl]trimethylammoniumchlorid (MAPTAC); Polyalkylenglykole und/oder Alkylpolyglykole handelt.

8. Mittel nach mindestens einem der Ansprüche 1 bis 7, **dadurch gekennzeichnet, dass** die Copolymerisation in Gegenwart mindestens eines polymeren Additivs G) erfolgt.

9. Mittel nach mindestens einem der Ansprüche 1 bis 8, **dadurch gekennzeichnet, dass** die Copolymere vernetzt sind.

10. Mittel nach mindestens einem der Ansprüche 1 bis 9, **dadurch gekennzeichnet, dass** die Copolymere durch Fällungspolymerisation in tert.-Butanol hergestellt werden.

11. Mittel nach mindestens einem der Ansprüche 1 bis 10, **dadurch gekennzeichnet, dass** die Copolymere wasserlöslich oder wasserquellbar sind.

12. Mittel nach mindestens einem der Ansprüche 1 bis 11, **dadurch gekennzeichnet, dass** sie, bezogen auf die fertigen Mittel, 0,01 bis 10 Gew.-% der Copolymere enthalten.

13. Mittel nach mindestens einem der Ansprüche 1 bis 12, **dadurch gekennzeichnet, dass** sie als Elektrolyte Salze, bevorzugt Natrium-, Kalium und/oder Ammoniumsalze, von ein- oder mehrwertigen Säuren und Basen, bevorzugt einwertige Säuren und/oder Basen, enthalten.

14. Mittel nach mindestens einem der Ansprüche 1 bis 12, **dadurch gekennzeichnet, dass** sie als Elektrolyte anorganische Salze, bevorzugt Ammonium- oder Metallsalze, von Halogeniden, Oxiden, Carbonaten, Hydrogencarbonaten, Phosphaten, Sulfaten und/oder Nitraten, insbesonders bevorzugt Natriumchlorid; und/oder organischen Salze, bevorzugt Ammonium- oder Metallsalze, der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure und/oder Galacturonsäure enthalten.

15. Mittel nach mindestens einem der Ansprüche 1 bis 14, **dadurch gekennzeichnet, dass** die Elektrolytkonzentration 0,01 bis 50 Gew.-%, bevorzugt 0,1 bis 50 Gew.-%, besonders bevorzugt 0,5 bis 10 Gew.-%, beträgt.

16. Mittel nach mindestens einem der Ansprüche 1 bis 14, **dadurch gekennzeichnet, dass** die Ionenstärke I im Bereich 10⁻⁵ mol/l bis 2 mol/l, besonders bevorzugt 10⁻² mol/l bis 1 mol/l, insbesondere bevorzugt 0,1 mol/l bis 0,75 mol/l, liegt.

17. Mittel nach mindestens einem der Ansprüche 1 bis 16, **dadurch gekennzeichnet, dass** es sich um Emulsionen, bevorzugt Öl-in-Wasser-Emulsionen, mit Viskositäten von 8000 mPas bis 50000 mPas handelt.

18. Mittel nach mindestens einem der Ansprüche 1 bis 16, **dadurch gekennzeichnet, dass** es sich dabei um wässrige Gele, bevorzugt enthaltend organische Lösemittel, mit Viskositäten von 15000 mPas bis 100000 mPas handelt.

19. Mittel nach mindestens einem der Ansprüche 1 bis 16, **dadurch gekennzeichnet, dass** es sich dabei um tensidhaltige Formulierungen, bevorzugt um Shampoos und Duschbäder, mit Viskositäten von 1000 mPas bis 15000 mPas handelt.

20. Mittel nach mindestens einem der Ansprüche 1 bis 20, **dadurch gekennzeichnet, dass** es sich dabei um Rinse-off Mittel handelt.

21. Mittel nach mindestens einem der Ansprüche 1 bis 20, **dadurch gekennzeichnet, dass** es sich dabei um Leave-on Produkte handelt.

22. Mittel nach mindestens einem der Ansprüche 1 bis 21, **dadurch gekennzeichnet, dass** es mindestens ein Copolymer enthält, welches durch Copolymerisation mindestens der Komponenten A), B) und F) erhältlich ist.

## Claims

1. An electrolyte-containing cosmetic, dermatological or pharmaceutical composition which comprises at least one copolymer obtainable by free-radical copolymerization of
A) acryloyldimethyltaurine and/or acryloyldimethyltaurates,
B) if desired, one or more further olefinically unsaturated, noncationic, optionally crosslinking comonomers which have at least one oxygen, nitrogen, sulfur or phosphorus atom and possess a molecular weight of less than 500 g/mol,
C) if desired, one or more olefinically unsaturated, cationic comonomers which have at least one oxygen, nitrogen, sulfur or phosphorus atom and possess a molecular weight of less than 500 g/mol,
D) if desired, one or more silicon-containing components capable of free-radical polymerization and having a functionality of at least one,
E) if desired, one or more fluorine-containing components capable of free-radical polymerization and having a functionality of at least one,
F) if desired, one or more olefinically mono- or polyunsaturated, optionally crosslinking macromonomers each possessing at least one oxygen, nitrogen, sulfur or phosphorus atom and having a number-average molecular weight of greater than or equal to 200 g/mol, the macromonomers not being a silicon-containing component D) or fluorine-containing component E), and where at least one macromonomer is a macromonomer of formula (IV) 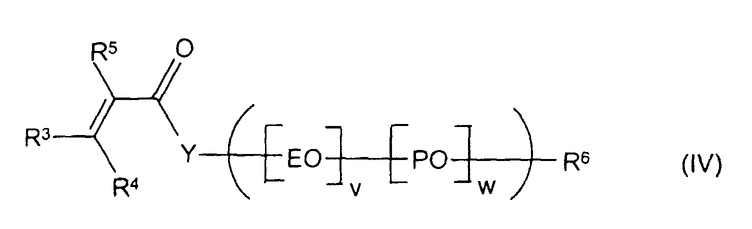 in which
R₃, R₄, R₅, and R₆ independently of one another are hydrogen or n-aliphatic, iso-aliphatic, olefinic, cycloaliphatic, arylaliphatic or aromatic (C₁-C₃₀) hydrocarbon radicals,
v and w independently of one another are from 0 to 500,
the sum of v and w is on average ≥1, and
Y is a bridge selected from -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH-, and -N(CH₃)-,
G) the copolymerization taking place if desired in the presence of at least one polymeric additive having number-average molecular weights of from 200 g/mol to 10⁹ g/mol,
H) with the proviso that component A) is copolymerized with at least one component selected from one of the groups D) to G),
and in which, based on the total mass of the copolymers, the amount of acryloyldimethyltaurine and/or acryloyldimethyltaurates is at least 0.1 % by weight.

2. A composition as claimed in claim 1, wherein the comonomers B) are unsaturated carboxylic acids, salts of unsaturated carboxylic acids, anhydrides of unsaturated carboxylic acids, esters of unsaturated carboxylic acids with aliphatic, olefinic, cycloaliphatic, arylaliphatic or aromatic alcohols having 1 to 22 carbon atoms, open-chain N-vinyl amides, cyclic N-vinyl amides having a ring size of from 3 to 9, amides of acrylic acid, amides of methacrylic acid, amides of substituted acrylic acids, amides of substituted methacrylic acids, 2-vinylpyridine, 4-vinylpyridine, vinyl acetate; styrene, acrylonitrile, vinyl chloride, vinylidene chloride, tetrafluoroethylene, vinylphosphonic acid or the esters or salts thereof, vinylsulfonic acid or the esters or salts thereof, allylphosphonic acid or the esters or salts thereof and/or methallylsulfonic acid or the esters or salts thereof.

3. A composition as claimed in claim 1 and/or 2, wherein the comonomers C) are
diallyldimethylammonium chloride (DADMAC),
[2-(methacryloyloxy)ethyl]trimethylammonium chloride (MAPTAC),
[2-(acryloyloxy)ethyl]trimethylammonium chloride,
[2-methacrylamidoethyl]trimethylammonium chloride,
[2-(acrylamido)ethyl]trimethylammonium chloride,
N-methyl-2-vinylpyridinium chloride,
N-methyl-4-vinylpyridinium chloride,
dimethylaminoethyl methacrylate,
dimethylaminopropylmethacrylamide,
methacryloylethyl N-oxide and/or
methacryloylethylbetaine.

4. A composition as claimed in at least one of claims 1 to 3, wherein the silicon-containing components D) are compounds of the formula (I)
R¹-Z-[(Si(R³R⁴)-O-)_{w}-(Si(R⁵R⁶)-O)ₓ-]-R² (I)
where
R¹ represents a vinyl, allyl, methallyl, methylvinyl, acryloyl, methacryloyl, crotonyl, senecionyl, itaconyl, maleyl, fumaryl or styryl radical;
Z is a chemical bridge, preferably selected from -O-, -((C₁-C₅₀)alkylene)-, -((C₆-C₃₀)arylene)-, -((C₅-C₈)cycloalkylene)-, -((C₁-C₅₀)alkenylene)-, -(polypropylene oxide)ₙ-, -(polyethylene oxide)ₒ-, (polypropylene oxide)ₙ(polyethylene oxide)ₒ-, where n and o independently of one another denote numbers from 0 to 200, and the distribution of the EO/PO units can be random or in the form of blocks,
((C₁-C₁₀)alkyl)-(Si(OCH₃)₂)- and -(Si(OCH₃)₂)-;
R³, R⁴, R⁵, and R⁶ independently of one another are -CH₃, -O-CH₃, -C₆H₅ or -O-C₆H₅;
w, x denote numbers from 0 to 500, it being necessary for either w or x to be greater than zero; and
R² is a saturated or unsaturated aliphatic, cycloaliphatic, arylaliphatic or aromatic radical having in each case 1 to 50 carbon atoms or a group of the formulae -OH, -NH₂, -N(CH₃)₂, -R⁷ or a group -Z-R¹, where Z and R¹ have the meanings mentioned above, and R⁷ is a group of the formula -O-Si(CH₃)₃, -O-Si(phenyl)₃, -O-Si(O-Si(CH₃)₃)₂CH₃) and -O-Si(O-Si(Ph)₃)₂Ph).

5. A composition as claimed in at least one of claims 1 to 4, wherein the fluorine-containing components E) are compounds of the formula (II)
R¹-Y-CᵣH₂ᵣCₛF₂ₛCF₃ (II)
where
R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a chemical bridge, preferably -O-, -C(O)-, -C(O)-O-, -S-, -O-CH₂-CH(O-)-CH₂OH, -O-CH₂-CH(OH)-CH₂-O-, -O-SO₂-O- , -O-S(O)-O-, -PH-, -P(CH₃)-, -PO₃-, -NH-, -N(CH₃)-, -O-(C₁-C₅₀)alkyl-O-, -O-phenyl-O-, -O-benzyl-O-, -O-(C₅-C₈)cycloalkyl-O-, -O-(C₁-C₅₀)alkenyl-O-, -O-(CH(CH₃)-CH₂-O)ₙ-, -O-(CH₂-CH₂-O)ₙ-, and -O-([CH-CH₂-O]ₙ-[CH₂-CH₂-O]ₘ)ₒ-, where n, m, and o independently of one another denote numbers from 0 to 200, and
r, s are stoichiometric coefficients which independently of one another are numbers between 0 and 200.

6. A composition as claimed in at least one of claims 1 to 5, wherein component F) comprises besides the macromonomers of formula (IV) additionally one or more macromonomers of formula (III)
R¹-Y-[(A)ᵥ-(B)_{w}-(C)ₓ-(D)_{z}]-R² (III)
where R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a bridging group, preferably -O-, -S-, -C(O)-, -C(O)-O-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH-, and -N(CH₃)-;
A, B, C, and D independently of one another are discrete chemical repeating units, preferably originating from acrylamide, methacrylamide, ethylene oxide, propylene oxide, AMPS, acrylic acid, methacrylic acid, methyl methacrylate, acrylonitrile, maleic acid, vinyl acetate, styrene, 1,3-butadiene, isoprene, isobutene, diethylacrylamide, and diisopropylacrylamide, more preferably ethylene oxide or propylene oxide;
v, w, x, and z independently of one another amount to from 0 to 500, preferably from 1 to 30, the sum of v, w, x, and z being on average ≥1; and
R² is a linear or branched aliphatic, olefinic, cycloaliphatic, arylaliphatic or aromatic (C₁-C₅₀) hydrocarbon radical, OH, -NH₂ or -N(CH₃)₂ or is [-Y-R¹].

7. A composition as claimed in at least one of claims 1 to 6, wherein the polymeric additives G) are homopolymers or copolymers of N-vinylformamide, N-vinylacetamide, N-vinylpyrrolidone, ethylene oxide, propylene oxide, acryloyldimethyltaurine, N-vinylcaprolactam, N-vinylmethylacetamide, acrylamide, acrylic acid, methacrylic acid, N-vinylmorpholide, hydroxymethyl methacrylate, diallyldimethylammonium chloride (DADMAC) and/or [2-(methacryloyloxy)ethyl]trimethylammonium chloride (MAPTAC); polyalkylene glycols and/or alkylpolyglycols.

8. A composition as claimed in at least one of claims 1 to 7, wherein the copolymerization takes place in the presence of at least one polymeric additive G).

9. A composition as claimed in at least one of claims 1 to 8, wherein the copolymers are crosslinked.

10. A composition as claimed in at least one of claims 1 to 9, wherein the copolymers are prepared by precipitation polymerization in tert-butanol.

11. A composition as claimed in at least one of claims 1 to 10, wherein the copolymers are water-soluble or water-swellable.

12. A composition as claimed in at least one of claims 1 to 11, which comprises, based on the finished composition, from 0.01 to 10% by weight of the copolymers.

13. A composition as claimed in at least one of claims 1 to 12, which comprises as electrolytes salts, preferably sodium, potassium and/or ammonium salts, of monovalent or polyvalent acids and bases, preferably monovalent acids and/or bases.

14. A composition as claimed in at least one of claims 1 to 12, which comprises as electrolytes inorganic salts, preferably ammonium salts or metal salts, of halides, oxides, carbonates, hydrogencarbonates, phosphates, sulfates and/or nitrates, more preferably sodium chloride; and/or organic salts, preferably ammonium salts or metal salts, of glycolic acid, lactic acid, citric acid, tartaric acid, mandelic acid, salicylic acid, ascorbic acid, pyruvic acid, fumaric acid, retinoic acid, sulfonic acids, benzoic acid, kojic acid, fruit acid, malic acid, gluconic acid and/or galacturonic acid.

15. A composition as claimed in at least one of claims 1 to 14, wherein the electrolyte concentration is from 0.01 to 50% by weight, preferably from 0.1 to 50% by weight, more preferably from 0.5 to 10% by weight.

16. A composition as claimed in at least one of claims 1 to 14, wherein the ionic strength I is in the range from 10⁻⁵ mol/l to 2 mol/l, more preferably from 10⁻² mol/l to 1 mol/l, very preferably from 0.1 mol/l to 0.75 mol/l.

17. A composition as claimed in at least one of claims 1 to 16, which is an emulsion, preferably an oil-in-water emulsion, having viscosities of from 8 000 mPas to 50 000 mPas.

18. A composition as claimed in at least one of claims 1 to 16, which is an aqueous gel, preferably comprising organic solvents, having viscosities of from 15 000 mPas to 100 000 mPas.

19. A composition as claimed in at least one of claims 1 to 16, which is a surfactant-containing formulation, preferably a shampoo or body wash, having viscosities of from 1 000 mPas to 15 000 mPas.

20. A composition as claimed in at least one of claims 1 to 19, which is a rinse-off composition.

21. A composition as claimed in at least one of claims 1 to 20, which is a leave-on product.

22. A composition as claimed in at least one of claims 1 to 21, which comprises at least one copolymer obtainable by copolymerizing at least components A), B), and F).

## Revendications

1. Agents pharmaceutiques, dermatologiques et cosmétiques contenant un électrolyte, **caractérisés en ce qu'**ils renferment au moins un copolymère que l'on peut obtenir par copolymérisation radicalaire
A) d'acide acryloyldiméthyltaurique et/ou d'acryloyldiméthyltaurates,
B) éventuellement d'un ou plusieurs autres comonomères à insaturation oléfinique, non cationiques, éventuellement réticulants, qui présentent au moins un atome d'oxygène, d'azote, de soufre ou de phosphore et possèdent une masse moléculaire inférieure à 500 g/mole,
C) éventuellement d'un ou plusieurs comonomères cationiques à insaturation oléfinique, qui présentent au moins un atome d'oxygène, d'azote, de soufre ou de phosphore et possèdent une masse moléculaire inférieure à 500 g/mole,
D) éventuellement d'un ou plusieurs composant(s) au moins monofonctionnels renfermant du silicium, aptes à la polymérisation radicalaire,
E) éventuellement d'un ou plusieurs composant(s) au moins monofonctionnels renfermant du fluor, aptes à la polymérisation radicalaire,
F) éventuellement d'un ou plusieurs macromonomères à une ou plusieurs insaturations oléfiniques, éventuellement réticulants, chacun possédant au moins un atome d'oxygène, d'azote, de soufre ou de phosphore et présentant une masse moléculaire moyenne en nombre supérieure ou égale à 200 g/mole, en ce qui concerne les macromonomères, il ne s'agit pas d'un composant contenant du silicium D) ou d'un composant contenant du fluor E), et au moins un macromonomère étant un macromonomère de formule de formule (IV) 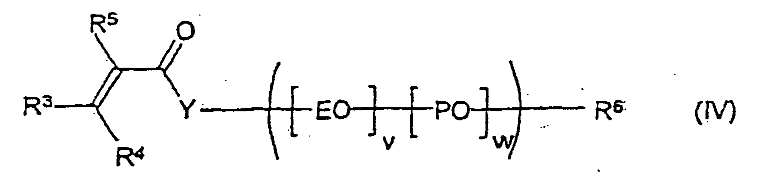 où
R₃, R₄, R₅ et R₆ indépendamment les uns des autres représentent un atome d'hydrogène ou des restes hydrocarbonés en C₁-C₃₀ n-aliphatiques, isoaliphatiques, oléfiniques, cycloaliphatiques, arylaliphatiques ou aromatiques,
v et w indépendamment l'un de l'autre vont de 0 à 500,
la somme de v et w est en moyenne ≥1, et
Y est une liaison prise parmi -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- et -N(CH₃)-,
G) la copolymérisation étant mise en oeuvre éventuellement en présence d'au moins un additif polymère ayant des masses moléculaires moyennes en nombre de 200 g/mole à 10⁹ g/mole,
H) à condition que le composant A) soit copolymérisé avec au moins un composant pris dans l'un des groupes D) à G),
et les teneurs en acide acryloyldiméthyltaurique ou en acryloyldiméthyltaurates, par rapport à la masse totale des copolymères, sont d'au moins 0,1 % en masse.

2. Agents selon la revendication 1, **caractérisés en ce que** les comonomères B) sont des acides carboxyliques insaturés, des sels d'acides carboxyliques insaturés, des anhydrides d'acides carboxyliques insaturés, des esters d'acides carboxyliques insaturés avec des alcools aliphatiques, oléfiniques, cycloaliphatiques, arylaliphatiques ou aromatiques présentant 1 à 22 atomes de carbone, des N-vinylamides à chaîne ouverte, des N-vinylamides cycliques ayant une taille de cycle de 3 à 9, des amides de l'acide acrylique, des amides de l'acide méthacrylique, des amides d'acides acryliques substitués, des amides d'acides méthacryliques substitués, la 2-vinylpyridine, la 4-vinylpyridine, l'acétate de vinyle ; le styrène, l'acrylonitrile, le chlorure de vinyle, le chlorure de vinylidène, le tétrafluoréthylène, l'acide vinylphosphonique ou ses esters ou sels, l'acide vinylsulfonique ou ses esters ou sels, l'acide allylphosphonique ou ses esters ou sels et/ou méthallylsulfonique ou ses esters ou sels.

3. Agents selon la revendication 1 et/ou 2, **caractérisés en ce que** les comonomères C) sont
le chlorure de diallyldiméthylammonium (DADMAC),
le chlorure de [2-(méthacryloyloxy)éthyl]triméthylammonium (MAPTAC),
le chlorure de [2-(acryloyloxy)éthyl]triméthylammonium,
le chlorure de [2-méthacrylamidoéthyl]triméthylammonium,
le chlorure de [2-(acrylamido)éthyl]triméthylammonium,
le chlorure de N-méthyl-2-vinylpyridinium,
le chlorure de N-méthyl-4-vinylpyridinium,
le méthacrylate de diméthylaminoéthyle,
le méthacrylamide de diméthylaminopropyle,
le N-oxyde de méthacryloyléthyle et/ou
le méthacryloyléthyl-bétaine.

4. Agents selon au moins l'une des revendications 1 à 3, **caractérisés en ce que** les composants contenant du silicium D) sont des composés de formule (I)
R¹-Z-[(Si(R³R⁴)-O-)_{w}-(Si(R⁵R⁶)-O)ₓ-]-R² (I)
R¹ représente un reste vinyle, allyle, méthallyle, méthylvinyle, acryle, méthacryle, crotonyle, sénécionyle, itaconyle, maléyle, fumaryle ou styryle ;
Z représente une liaison chimique prise de préférence parmi -O-, -(alkylène en C₁-C₅₀)-, -(arylène en C₆-C₃₀)-, - (cycloalkylène en C₅-C₈)-, -(alcénylène en C₁-C₅₀)-, -(polyoxypropylène)ₒ-, -(polyoxyéthylène)ₒ-, -(polyoxypropylène)ₙ-(polyoxyéthylène)ₒ-, n et o indépendamment l'un de l'autre étant des nombres de 0 à 200 et la distribution des unités OE/OP peut être statistique ou à blocs, (alkyle en C₁-C₁₀)-(Si(OCH₃)₂)- et -(Si(OCH₃)₂)- ;
R³, R⁴, R⁵ et R⁶ indépendamment les uns des autres représentent un groupe -CH₃, -O-CH₃, -C₆H₅ ou -O-C₆H₅ ;
w, x vont de 0 à 500, soit w soit x devant être supérieur à zéro, et
R² représente un reste aliphatique, cycloaliphatique, arylaliphatique ou aromatique saturé ou insaturé chacun ayant 1 à 50 atomes de carbone ou un groupe de formules -OH, -NH₂, -N(CH₃)₂, -R⁷ ou un groupe de formule -Z-R¹, où Z et R¹ possèdent les significations données ci-dessus et R⁷ représente un groupe de formules -O-Si(CH₃)₃, -O-Si(phényle)₃, -O-Si (O-Si (CH₃)₃)₂CH₃) et -O-Si (O-Si (Ph)₃)₂Ph).

5. Agents selon au moins l'une des revendications 1 à 4, **caractérisés en ce que** les composants fluorés E) sont des composés de formule (II)
R¹-Y-CᵣH₂ᵣCₛF₂ₛCF₃ (II)
où
R¹ représente une fonction apte à la polymérisation prise dans le groupe comprenant des composés à insaturation vinylique, de préférence un reste vinyle, allyle, méthallyle, méthylvinyle, acryle, méthacryle, crotonyle, sénécionyle, itaconyle, maléyle, fumaryle ou styryle ;
Y représente une liaison chimique de préférence -O-, -C(O)-, -C(O)-O-, -S-, -O-CH₂-CH(O-)-CH₂OH, -O-CH₂-CH(OH)-CH₂-O-, -O-SO₂-O-, -O-S-(O)-O-, -PH-, -P(CH₃)-, -PO₃-, -NH-, -N(CH₃)-, -O-(alkyl en C₁-C₅₀)-O-, -O-phényl-O-, -O-benzyl-O-, -O-(cycloalkyl en C₅-C₈)-O-, -O-(alcényl en C₁-C₅₀)-O-, -O- (CH (CH₃) -CH₂-O)ₙ-, -O- (CH₂-CH₂-O)ₙ- et -O-([CH-CH₂-O]ₙ-[CH₂-CH₂-O]ₘ)ₒ, n, m et o indépendamment l'un de l'autre étant des nombres de 0 à 200, et
r, s représentent des coefficients stoechiométriques qui, indépendamment l'un de l'autre, sont des nombres compris entre 0 et 200.

6. Agents selon au moins l'une des revendications 1 à 5, **caractérisés en ce que** le composant F) renferme de plus, au côté des macromonomères de formule (IV), un ou plusieurs macromonomères de formule (III),
R¹-Y-[(A)ᵥ-(B)_{w}-(C)ₓ-(D)_{z}]-R² (III)
où
R¹ représente une fonction apte à la polymérisation prise dans le groupe des composés à insaturation vinylique, de préférence un reste vinyle, allyle, méthallyle, méthylvinyle, acryle, méthacryle, crotonyle, sénécionyle, itaconyle, maléyle, fumaryle ou styryle ;
Y représente un groupe de liaison, de préférence -O-, -S-, -C(O)-, -C(O)-O-, -O-CH₂-CH(O-)-CH₂OH, -O-CH₂-CH (OH) -CH₂O-, -O-SO₂-O-, -O-SO₂-O-, -O-SO-O-, -PH-, -P(CH₃)-, -PO₃-, -NH- et -N(CH₃)- ;
A, B, C et D indépendamment les uns des autres représentent des unités chimiques récurrentes discrètes, de préférence résultant de l'acrylamide, du méthacrylamide, de l'oxyde d'éthylène, de l'oxyde de propylène, de l'AMPS, de l'acide acrylique, de l'acide méthacrylique, du méthacrylate de méthyle, de l'acrylonitrile, de l'acide maléique, de l'acétate de vinyle, du styrène, du 1,3-butadiène, de l'isoprène, de l'isobutène, du diéthylacrylamide et du diisopropylacrylamide, de manière particulièrement préférée de l'oxyde d'éthylène, de l'oxyde de propylène ;
v, w, x et z indépendamment les uns des autres vont de 0 à 500, de préférence de 1 à 30, la somme de v, w, x et z est en moyenne ≥1 ; et
R² représente un reste hydrocarboné en C₁-C₅₀ linéaire ou ramifié aliphatique, oléfinique, cycloaliphatique, arylaliphatique ou aromatique, OH, -NH₂ ou -N(CH₃)₂ ou est égal à [-Y-R¹].

7. Agents selon au moins l'une des revendications 1 à 6, **caractérisés en ce que** les additifs polymères G) sont des homo- ou copolymères pris dans le groupe comprenant le N-vinylformamide, le N-vinylacétamide, la N-vinylpyrrolidone, l'oxyde d'éthylène, l'oxyde de propylène, l'acide acryloyldiméthyltaurique, le N-vinylcaprolactame, le N-vinylméthylacétamide, l'acrylamide, l'acide acrylique, l'acide méthacrylique, le N-vinylmorpholide, le méthacrylate d'hydroxyméthyle, le chlorure de diallyldiméthylammonium (DADMAC) et/ou le chlorure de [2-(méthacryloyloxy)éthyl]triméthylammonium (MAPTAC) ; des polyalkylèneglycols et/ou alkylpolyglykols.

8. Agents selon au moins l'une des revendications 1 à 7, **caractérisés en ce que** la copolymérisation est mise en oeuvre en présence d'au moins un additif polymère G).

9. Agents selon au moins l'une des revendications 1 à 8, **caractérisés en ce que** les copolymères sont réticulés.

10. Agents selon au moins l'une des revendications 1 à 9, **caractérisés en ce qu'**on prépare les copolymères par polymérisation par précipitation dans du tert.-butanol.

11. Agents selon au moins l'une des revendications 1 à 10, **caractérisés en ce que** les copolymères sont hydrosolubles ou hydrogonflables.

12. Agents selon au moins l'une des revendications 1 à 11, **caractérisés en ce qu'**ils renferment, par rapport à l'agent fini, de 0,01 à 10 % en masse des copolymères.

13. Agents selon au moins l'une des revendications 1 à 12, **caractérisés en ce qu'**ils renferment en tant qu'électrolytes des sels, de préférence des sels de sodium, de potassium et/ou d'ammonium, d'acides et de bases mono- ou multivalents, de préférence d'acides et/ou de bases monovalents.

14. Agents selon au moins l'une des revendications 1 à 12, **caractérisés en ce qu'**ils renferment en tant qu'électrolytes des sels inorganiques, de préférence des sels d'ammonium ou métalliques, d'halogénures, d'oxydes, de carbonates, de bicarbonates, de phosphates, de sulfates et/ou nitrates, de manière particulièrement préférée le chlorure de sodium ; et/ou des sels organiques, de préférence d'ammonium ou métalliques, d'acide glycolique, d'acide lactique, d'acide citrique, d'acide tartrique, d'acide mandélique, d'acide salicylique, d'acide ascorbique, d'acide pyruvique, d'acide fumarique, d'acide rétinoïque, d'acides sulfoniques, d'acide benzoïque, d'acide cojique, d'acides de fruits, d'acide malique, d'acide gluconique et/ou d'acide galacturonique.

15. Agents selon au moins l'une des revendications 1 à 14, **caractérisés en ce que** la concentration en électrolyte est de 0,01 à 50 % en masse, de préférence de 0,1 à 50 % en masse, de manière particulièrement préférée de 0,5 à 10 % en masse.

16. Agents selon au moins l'une des revendications 1 à 14, **caractérisés en ce que** la force ionique 1 se trouve dans le domaine de 10⁻⁵ mole/l à 2 mole/l, en manière particulièrement préférée de 10⁻² mole/l à 1 mole/l, de manière particulière de 0,1 mole/l à 0,75 mole/l.

17. Agents selon au moins l'une des revendications 1 à 16, **caractérisés en ce qu'**il s'agit d'émulsions, de préférence d'émulsions huile-dans-l'eau ayant des viscosités de 8000 mPas à 50000 mPas.

18. Agents selon au moins l'une des revendications 1 à 16, **caractérisés en ce qu'**il s'agit de gel aqueux, de préférence renfermant des solvants organiques, ayant des viscosités de 15000 mPas à 100000 mPas.

19. Agents selon au moins l'une des revendications 1 à 16, **caractérisés en ce qu'**il s'agit de formulations renfermant des agents de surface, de préférence de shampoings et de bains de douche, ayant des viscosités de 1000 mPas à 15000 mPas.

20. Agents selon au moins l'une des revendications 1 à 20, **caractérisés en ce qu'**il s'agit de produits qui se rincent à l'eau ("rince-off").

21. Agents selon au moins l'une des revendications 1 à 20, **caractérisés en ce qu'**il s'agit de produits que l'on laisse poser ("leave-on").

22. Agent selon au moins l'une des revendications 1 à 21, **caractérisé en ce qu'**il renferme au moins un copolymère que l'on peut obtenir par copolymérisation d'au moins des composants A), B) et F).
